# EUROPEAN PATENT SPECIFICATION

(11) **EP 1 065 206 B1**
(45) Date of publication and mention of the grant of the patent: **09.10.2002**
(21) Application number: 00305355.0
(22) Date of filing: 26.06.2000
(51) Int. Cl.: C07D 401/14, C07D 403/06, C07D 405/14, C07D 409/14, C07D 403/14, A61K 31/41, A61P 29/00

(54) **Tetrazolylalkyl indole compounds as anti-inflammatory and analgesic agents**
Tetrazolylalkyl-Indolderivate als entzündungshemmende und analgetische Mittel
Dérivés de tétrazolylalkyl-indole comme agents anti-inflammatoires et analgésiques

(30) Priority: 02.07.1999 WO PCT/IB99/01244
(43) Date of publication of application: 03.01.2001
(73) Proprietor: PFIZER INC., New York, N.Y. 10017 (US)
(72) Inventor: Nakao, Kazunari, Central Research Nagoya, Chita-gun, Aichi-ken 470-2393 (JP); Stevens, Rodney William, Central Research Nagoya, Chita-gun, Aichi-ken 470-2393 (JP)
(74) Representative: Simpson, Alison Elizabeth Fraser

(56) References cited:
- EP-A- 0 275 667
- WO-A-99/05104
- WO-A-99/35130

## Description

### Technical Field

This invention relates to tetrazolylalkyl-substituted indoles as pharmaceutical agents. This invention specifically relates to compounds, compositions and methods for the treatment or alleviation of pain, inflammation, other inflammation-associated disorders such as arthritis, and the like in manmalia such as humans, dogs and cats.

### Background Art

Nonsteroidal antiinflammatory drugs (NSAIDs) are widely used in treating pain and the signs and symptoms of arthritis because of their analgesic and anti-inflammatory activity. It is accepted that common NSAIDs work by blocking the activity of cyclooxygenase (COX), also known as prostaglandin G/H synthase (PGHS), the enzyme that converts arachidonic acid into prostanoids. Prostaglandins, especially prostaglandin E₂ (PGE₂), which is the predominant eicosanoid detected in inflammation conditions, are mediators of pain, fever and other symptoms associated with inflammation. Inhibition of the biosynthesis of prostaglandins has been a therapeutic target of anti-inflammatory drug discovery. The therapeutic use of conventional NSAIDs is, however, limited due to drug associated side effects, including life threatening ulceration and renal toxicity. An alternative to NSAIDs is the use of corticosteriods, however, long term therapy can also result in severe side effects.

Recently, two forms of COX were identified, a constitutive isoform (COX-1) and an inducible isoform (COX-2) of which expression is upregulated at sites of inflammation (Vane, J. R.; Mitchell, J. A.; Appleton, I.; Tomlinson, A.; Bishop-Bailey, D.; Croxtoll, J.;Willoughby, D. A. *Proc. Natl. Acad. Sci. USA,* **1994,** *91*, 2046). COX-1 is thought to play a physiological role and to be responsible for gastrointestinal and renal protection. On the other hand, COX-2 appears to play a pathological role and to be the predominant isoform present in inflammation conditions. A pathological role for prostaglandins has been implicated in a number of human disease states including rheumatoid and osteoarthritis, pyrexia, asthma, bone resorption, cardiovascular diseases, nephrotoxicity, atherosclerosis, hypotension, shock, pain, cancer, and Alzheimer disease. The NSAIDs currently on market inhibit both isoforms of COX with little variation for selectivity, explaining their beneficial (inhibition of COX-2) and deleterious effects (inhibition of COX-1). It is believed that compounds that would selectively inhibit the biosynthesis of prostaglandins by intervention of the induction phase of the inducible enzyme cyclooxygenase-2 and/or by intervention of the activity of the enzyme cyclooxygenase-2 on arachidonic acid would provide alternate therapy to the use of NSAIDs or corticosteriods in that such compounds would exert anti-inflammatory effects without the adverse side effects associated with COX-1 inhibition.

A variety of indole compounds are known and are disclosed in several patent publications. International Publication Number WO 96/32379 discloses N-substituted indole compounds as cGMP-PDE Inhibitors. International Publication Numbers WO 96/37467, WO 96/37469, UK Patent Publication GB 2283745 A and US Patent Number 5510368 disclose 2-methyl-N-substituted indole compounds as cyclooxygenase-2 Inhibitors. Also, a variety of indole compounds are disclosed as agents for controlling underwater fouling organisms in European Patent Publication Number 0 556 949 A2. International Publication Number WO 97/09308 discloses indole compounds as neuropeptide receptor antagonists. International Publication Number WO 99/05104 discloses 3-amino-substituted indole compounds. In addition, Sci. Pharm. **64**, 577 (1996) describes a process for preparing a 2-ester-substituted indoline.

### Brief Disclosure of the Invention

The present invention provides a compound of the following formula: 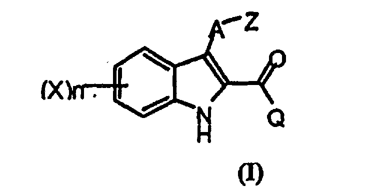 or the pharmaceutically acceptable salts thereof wherein
- **Z**: is tetrazolyl optionally substituted with C₁₋₄ alkyl or halosubstituted C₁₋₄ alkyl;
- **A**: is C₁₋₆ alkylene;
- **Q**: is a cyclic group selected from:
(a) phenyl;
(b) a partially saturated, fully saturated or fully unsaturated five to six membered monocyclic group having one to four heteroatoms selected independently from N, 0 and S; and
(c) a bicyclic group consisting of two fused partially saturated, fully saturated or fully unsaturated five to eight membered rings independently and optionally having one to four heteroatoms selected independently from N, O and S,
said cyclic groups (a), (b) and (c) being independently and optionally substituted with one to five substituents independently selected from halogen, C₁₋₄ alkyl, halosubstituted C₁₋₄ alkyl, OH, *C*₁₋₄ alkoxy, halosubstituted C₁₋₄ alkoxy, C₁₋₄ alkylthio, NO₂, NH₂, mono- or di-(C₁₋₄ alkyl)amino, CN, hydroxy-C₁₋₄ alkyl and C₁₋₄ alkoxy-C₁₋₄ alkyl;
- **X**: is independently selected from halogen, C₁₋₄ alkyl, halosubstituted C₁₋₄ alkyl, OH, C₁₋₄ alkoxy, halosubstituted C₁₋₄ alkoxy, C₁₋₄ alkylthio, NO₂, NH₂ and mono- or di-(C₁₋₄ alkyl)amino and CN; and
- **n**: is 0, 1, 2, 3 or 4;

The indole compounds of the present invention exhibit inhibition of COX activity. Preferably compounds of this invention exhibit inhibitory activity against COX-2, with more preferable compounds having COX-2 selectivity.

Accordingly, the present invention also provides a pharmaceutical composition, useful for the treatment of a medical condition in which prostaglandins are implicated as pathogens, which comprises a compound of the formula (I) and the pharmaceutically acceptable salts thereof.

Further, the present invention provides a method for the treatment of a medical condition in which prostaglandins are implicated as pathogens, in a mammalian subject, which comprises administering to said subject a therapeutically effective amount of said pharmaceutical composition.

The medical conditions in which prostaglandins are implicated as pathogens, include the relief of pain, fever and inflammation of a variety of conditions including rheumatic fever, symptoms associated with influenza or other viral infections, common cold, low back and neck pain, dysmenorrhea, headache, toothache, sprains and strains, myositis, neuralgia, synovitis, arthritis including rheumatoid arthritis, degenerative joint disease (osteoarthritis), gout, ankylosing spondylitis, systemic lumpus erythematosus and juvenile arthritis, bursitis, burns, injuries following surgical and dental procedures.

The compounds and pharmaceutical composition of this invention may inhibit cellular neoplastic transformations and metastatic tumor growth and thus may be used in the treatment and/or prevention of cancers in the colon, breast, skin, esophagus, stomach, urinary bladder, lung and liver. The compounds and pharmaceutical composition of this invention were used in the treatment and/or prevention of cyclooxygenase-mediated proliferation disorders such as which occur in diabetic retinopathy and tumor angiogenesis.

The compounds and pharmaceutical composition of this invention may inhibit prostaniod-induced smooth muscle contraction by preventing the synthesis of contractile prostanoids, and thus may be of use in the treatment of dysmenorrhea, premature labor, asthma and eosinophil related disorders and in the treatment of neurodegenerative diseases such as Alzheimer's and Parkinson's disease, and for the treatment of bone loss (treatment of osteoarthritis), stroke, seizures, migraine, multiple sclevosis, AIDS and encephaloathy.

By virtue of the COX-2 activity and/or specificity for COX-2 over COX-1, such compounds will prove useful as an alternative to conventional NSAIDs particularly where such NSAIDs may be contra-indicated such as in patients with ulcers (such as peptic ulcers and gastric ulcers), gastritis, regional enterotis, ulcerative colitis, diverticulitis or with a recurrent history of GI lesions, GI bleeding, coagulation disorders including anemia such as hypoprothrombinemia, haemophilia and other bleeding problems; kidney disease; prior to surgery of taking of anticoagulants.

### Detailed Disclosure of the Invention

As used herein, **"halo"** is fluoro, chloro, bromo or iodo.

As used herein, the term **"alkyl"** means straight or branched chain saturated hydrocarbons wherein a hydrogen atom is removed from the terminal carbon. Exampls of such groups are methyl, ethyl, *n*-propyl, isopropyl, *n*-butyl, isobutyl, *sec*-butyl and *tert*-butyl.

As used herein, the term **"alkylene"** means straight or branched chain saturated hydrocarbons wherein a hydrogen atom is removed from each of the terminal carbons. Examples of such groups are methylene, ethylene, propylene, butylene, pentylene and hexylene.

As used herein, the term **"alkoxy"** means -O-alkyl such as methoxy, ethoxy, *n*-propoxy, isopropoxy, *n*-butoxy, isobutoxy, *sec*-butoxy and *tert*-butoxy.

As used herein, the term **"alkylthio"** means -S-alkyl such as methylthio, ethylthio, *n*-propylthio, isopropylthio, *n*-butylthio, isobutylthio, *sec*-butylthio and *tert*-butylthio.

As used herein, the term **"mono-alkylamino"** means mono-alkyl substituted amino such as methylamino, ethylamino, n-propylamino, isopropylamino, n-butylamino, isobutylamino, *sec*-butylamino and *tert*-butylamino.

As used herein, the term "**di-alkylamino**" means di-alkyl substituted amino such as dimethylamino, diethylamino, dipropylamino, N-methyl-N-ethylamino, N-methyl-N-propylamino, N-methyl-N-butylamino and N-ethyl-N-propylamino. As used herein, the term "**halosubstituted alkyl**" means an alkyl radical as described above substituted with one or more halogens, such as chloromethyl, dichloromethyl, fluoromethyl, difluoromethyl, trifluoromethyl and 2,2,2-trichloroethyl.

As used herein, the term "**halosubstituted alkoxy"** means an alkoxy radical as described above substituted with one or more halogens, such as chloromethoxy, dichloromethoxy, fluoromethoxy, difluoromethoxy, trifluoromethoxy and 2,2,2-trichloroethoxy.

As used herein, the term "**alkoxyalkyl**" means an alkyl radical as described above substituted with alkoxy as described above, such as methoxymethyl, ethoxymethyl, propoxymethyl, methoxyethyl, ethoxyethyl, propoxyethyl, methoxyethyl, ethoxyethyl and propoxyethyl.

Examples of **"partially saturated, fully saturated or fully unsaturated five membered monocyclic group having one to four heteroatoms selected independently from N, O and S",** are furyl, thienyl, 2H-pyrrolyl, 3H-pyrrolyl, pyrrolyl, 2-pyrrolinyl, 3-pyrrolinyl, pyrrolidinyl, 1,3-dioxolanyl, oxazolyl, thiazolyl, imidazolyl, 2H-imidazolyl, 2-imidazolinyl, imidazolidinyl, pyrazolyl, 2-pyrazolinyl, pyrazolidinyl, isoxazolyl, isothiazolyl, 1,2-dithiolyl, 1,3-dithiolyl, 3H-1,2-oxathiolyl, 1,2,3-oxadiazolyl, 1,2,4-oxadiazolyl, 1,2,5-oxadiazolyl, 1,3,4-oxadiazolyl, 1,2,3-triazolyl, 1,2,4-triazolyl, 1,3,4-thiadiazolyl, 1,2,3,4-oxatriazolyl, 1,2,3,5-oxatriazolyl, 3H-1,2,3-dioxazolyl, 1,2,4-dioxazolyl, 1,3,2-dioxazolyl, 1,3,4-dioxazolyl, 5H-1,2,5-oxathiazolyl and 1,3-oxathiolyl.

Examples of **"partially saturated, fully saturated or fully unsaturated six membered monocyclic group having one to four heteroatoms selected independently from N, O and S",** are 2H-pyranyl, 4H-pyranyl, pyridinyl, piperidinyl, 1,2-dioxinyl, 1,3-dioxinyl, 1,4-dioxanyl, morpholinyl, 1,4-dithianyl, thiomorpholinyl, pyridazinyl, pyrimidinyl, pyrazinyl, piperazinyl, 1,3,5-triazinyl, 1,2,4-triazinyl, 1,2,3-triazinyl, 1,3,5-trithianyl, 4H-1,2-oxazinyl, 2H-1,3-oxazinyl, 6H-1,3-oxazinyl, 6H-1,2-oxazinyl, 1,4-oxazinyl, 2H-1,2-oxazinyl, 4H-1,4-oxazinyl, 1,2,5-oxathiazinyl, 1,4-oxazinyl, o-isoxazinyl, p-isoxazinyl, 1,2,5-oxathiazinyl, 1,2,6-oxathiazinyl, 1,4,2-oxadiazinyl and 1,3,5,2-oxadiazinyl.

Examples of **"bicyclic groups consisting of two fused partially saturated, fully saturated or fully unsaturated five or eight membered rings independently and optionally having one to four heteroatoms selected independently from N, O and S",** are indolizinyl, indolyl, isoindolyl, 3H-indolyl, 1H-isoindolyl, indolinyl, isoindolinyl, cyclopenta[b]pyridinyl, pyrano[3,4-b]pyrrolyl, benzofuryl, isobenzofuryl, benzo[b]thienyl, benzo[c]thienyl, 1H-indazolyl, indoxazinyl, benzoxazolyl, anthranilyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, indenyl, isoindenyl, naphtyl, tetralinyl, decalinyl, 2H-1-benzopyranyl, pyrido[3,4-b]pyridinyl, pyrido[3,2-b]pyridinyl, pyrido[4,3-b]pyridinyl, 2H-1,3-benzoxazinyl, 2H-1,4-benzoxazinyl, 1H-2,3-benzoxazinyl, 4H-3,1-benzoxazinyl, 2H-1,2-benzoxazinyl, 4H-1,4-benzoxazinyl, 5,6,7,8,-tetrahydroisoquinolinyl, 2,3-dihydro-1,4-benzodioxinyl, 2,3-dihydro-1-benzofuranyl, 1,3-benzodioxolyl, indanyl, 1,2,3,4-tetrahydroquinolinyl, 2,3-dihydro-1H-indolyl, 1,2,3,4-tetrahydroisoquinolinyl, chromanyl, isochromanyl, 2,3-dihydrofuro[3,2-c]pyridinyl, 1,2,3,4-tetrahydropyrido[3,4-b]pyrazinyl, 1,2,3,4-tetrahydro[2,6]naphthyridinyl, 3,4-dihydro-1H-pyrano[3,4-c]pyridinyl, 1,2,3,4-tetrahydro[1,6]naphthyridinyl, 3,4-dihydro-2H-pyrano[3,2-c]pyridinyl, 1,2,3,4-tetrahydro[1,7]naphthyridinyl, 1,2,3,4-tetrahydro[2,7]naphthyridinyl, 3,4-dihydro-1H-pyrano[4,3-c]pyridinyl, 2,3-dihydro-1H-pyrrolo[3,2-c]pyridinyl, 2,3-dihydro-1H-pyrrolo[2,3-c]pyridinyl, 2,3-dihydrofuro[2,3-c]pyridinyl, 2,3-dihydro-1H-pyrrolo[3,4-c]pyridinyl, 1,3-dihydrofuro[3,4-c]pyridinyl, [1,3]dioxolo[4,5-c]pyridinyl, 3,4-dihydro-2H-pyrano[2,3-c]pyridinyl, 1,2,3,4-tetrahydroquinoxalinyl, 2,3-dihydro-1-benzothienyl, 1,3-dihydro-2-benzothienyl, 1,3-benzodithiolyl, 2,3-dihydrothieno[2,3-cJpyridinyl, 1,3-dihydrothieno[3,4-c]pyridinyl, [1,3]dithiolo[4,5-c]pyridinyl, thiochromanyl, 3,4-dihydro-1H-isothiochromenyl and 2,3-dihydro-1,4-benzodithiinyl.

Preferred compounds of this invention are those of the formula (I) wherein
- **Z**: is tetrazolyl optionally substituted with C₁₋₃ alkyl or halosubstituted C₁₋₃ alkyl; **A** is C₁₋₅ alkylene; **Q** is a cyclic group selected from:
(a) phenyl;
(b) a partially saturated, fully saturated or fully unsaturated five to six membered monocyclic group having one to three heteroatoms selected independently from N, O and S; and
(c) a bicyclic group consisting of two fused partially saturated, fully saturated or fully unsaturated five or six membered rings independently and optionally having one to three heteroatoms selected independently from N, O and S,
said cyclic groups (a), (b) and (c) being independently and optionally-substituted with one, two or three substituents independently selected from halogen, C₁₋₄ alkyl, halosubstituted C₁₋₄ alkyl, C₁₋₄ alkoxy, halosubstituted C₁₋₄ alkoxy and C₁₋₄ alkoxy-C₁₋ ₄ alkyl;
- **X**: is independently selected from halogen, C₁₋₄ alkyl, halosubstituted C₁₋₄ alkyl, OH and C₁₋₄ alkoxy; and n is 0, 1, 2 or 3.

Further preferred compounds of this invention are those of the formula (I) wherein **Z** is tetrazolyl; **A** is C₁₋₄ alkylene; **Q** is selected from the following groups:
(a) phenyl;
(b) a five membered monocyclic gourp selected from furyl, thienyl, pyrrolyl, pyrrolinyl, pyrrolidinyl, dioxolanyl, oxazolyl, thiazolyl, imidazolyl, imidazolinyl, imidazolidinyl, pyrazolyl, pyrazolinyl, pyrazolidinyl, isoxazolyl, isothiazolyl, dithiolyl, oxathiolyl, oxadiazolyl, thiadiazolyl, oxatriazolyl, dioxazolyl, oxathiazolyl and oxathiolyl, and a six membered monocyclic group selected from pyranyl, pyridinyl, piperidinyl, dioxinyl, dioxanyl, morpholinyl, dithianyl, thiomorpholinyl, pyridazinyl, pyrimidinyl, pyrazinyl, piperazinyl, triazinyl, triazinyl, trithianyl, oxazinyl, oxathiazinyl, isoxazinyl and oxadiazinyl.; and
(c) a bicyclic group selected from indolyl, isoindolyl, indolinyl, benzofuryl, dihydrobenzofuranyl, isobenzofuryl, benzothienyl, indazolyl, indoxazinyl, benzoxazolyl, anthranilyl, benzimidazolyl, benzthiazolyl, purinyl, quinolizinyl, quinolinyl, isoquinolinyl, tetrahydroquinolinyl, tetrahydroisoquinolinyl, cinnolinyl, phthalazinyl, quinazolinyl, quinoxalinyl, naphthyridinyl, pteridinyl, indenyl, isoindenyl, naphtyl, tetralinyl, decalinyl, benzopyranyl, pyridopyridinyl, benzoxazinyl, dihydrobenzodioxinyl, benzodioxolyl, indanyl, cyclopentapyridinyl, pyranopyrrolyl, chromanyl and isochromanyl;
said cyclic groups (a), (b) and (c) being independently and optionally substituted with one, two or three substituents independently selected from halogen, C₁₋₃ alkyl, halosubstituted C₁₋₃ alkyl, C₁₋₃ alkoxy, halosubstituted C₁₋₃ alkoxy and C₁₋₃ alkoxy-C₁₋₃ alkyl;
**X** is independently selected from halogen, C₁₋₄ alkyl and halosubstituted C₁₋₄ alkyl; and
**n** is 0,1 or 2.

Further preferred compounds of this invention are those of the formula (I) wherein **Z** is tetrazolyl; **A** is methylene; **Q** is a cyclic group selected from pyridinyl, phenyl, furyl, thienyl, imidazolyl, quinolinyl and isoquinolinyl, and the cyclic group being optionally substituted with methyl, ethyl, chloro, trifluoromethyl, methoxy or methoxymethyl: X is chloro, fluoro, methyl or trifluoromethyl; and **n** is 0 or 1.

Further preferred compounds of this invention are those of the formula (I) wherein **Z** is 1,2,3,4-tetrazolyl; **Q** is 2- or 4-pyridinyl, 3-, 4-, 5- or 6-methyl-2-pyridinyl, 4-ethyl-2-pyridinyl, 4- or 5-chloro-2-pyridinyl, 5-trifuloromethyl-2-pyridinyl, 4-methoxy-2-pyridinyl, 4-chlorophenyl, 3-methoxymethyl-2-furyl, 2-thienyl, 5-methyl-2-thienyl, 1-methyl-2-imidazolyl, 3-isoquinolinyl or 2-quinolinyl; and **X** is 6-chloro, 5-chloro, 6-trifluoromethyl, 5-trifluoromethyl, 5-fluoro, 6-fluoro or 6-methyl; and **n** is 0 or 1.

Preferred individual compounds of this invention are:
[6-chloro-3-(1H-1,2,3,4-tetrazol-5-ylmethyl)-1H-indol-2-yl](4-methyl-2-pyridinyl)-methanone;
{6-chloro-3-[(2-methyl-2H-1,2,3,4-tetrazol-5-yl)methyl]-1H-indol-2-yl}(4-methyl-2-pyridinyl)methanone;
6-chloro-3-[(1-methyl-1H-1,2,3,4-tetrazol-5-yl)methyl]-1H-indol-2-yl}(4-methyl-2-pyridinyl)methanone;
[6-chloro-3-(1H-1,2,3,4-tetrazol-5-ylmethyl)-1H-indol-2-yl](3-methyl-2-pyridinyl)-methanone;
[6-chloro-3-(1H-1,2,3,4-tetrazol-5-ylmethyl)-1H-indol-2-yl](5-methyl-2-pyridinyl)-methanone;
[6-chloro-3-(1H-1,2,3,4-tetrazol-5-ylmethyl)-1H-indol-2-yl](6-methyl-2-pyridinyl)-methanone;
[6-chloro-3-(1H-1,2,3,4-tetrazol-5-ylmethyl)-1H-indol-2-yl](4-ethyl-2-pyridinyl)-methanone;
[6-chloro-3-(1H-1,2,3,4-tetrazol-5-ylmethyl)-1H-indol-2-yl](2-pyridinyl)methanone;
[6-chloro-3-(1H-1,2,3,4-tetrazol-5-yImethyl)-1H-indol-2-yl](4-chloro-2-pyridinyI)-methanone;
[6-chloro-3-(1H-1,2,3,4-tetrazol-5-ylmethyl)-1H-indol-2-yl](5-chloro-2-pyridinyl)-methanone;
[6-chloro-3-(1H-1,2,3,4-tetrazol-5-ylmethyl)-1H-indol-2-yl](5-trifluoromethyl-2-pyridinyl)methanone;
[6-chloro-3-(1H-1,2,3,4-tetrazol-5-ylmethyl)-1H-indol-2-yl](4-methoxy-2-pyridinyl)-methanone;
[6-chloro-3-(1H-1,2,3,4-tetrazol-5-ylmethyl)-1H-indol-2-yl](4-pyridinyl)methanone;
[6-chloro-3-(1H-1,2,3,4-tetrazol-5-ylmethyl)-1H-indol-2-yl](4-chlorophenyl)-methanone;
[6-chloro-3-(1H-1,2,3,4-tetrazol-5-ylmethyl)-1H-indol-2-yl](3-methoxymethyl-2-furyl)methanone;
[6-chloro-3-(1H-1,2,3,4-tetrazol-5-ylmethyl)-1H-indol-2-yl](2-thienyl)methanone;
[6-chloro-3-(1H-1,2,3,4-tetrazol-5-ylmethyl)-1H-indol-2-yl](5-methyl-2-thienyl)-methanone;
{6-chloro-3-[(1-methyl-1H-1,2,3,4-tetrazol-5-yl)methyl]-1H-indol-2-yl}(5-methyl-2-thienyl)methanone;
{6-chloro-3-[(2-methyl-2H-1,2,3,4-tetrazol-5-yl)methyl]-1H-indol-2-yl}(5-methyl-2-thienyl)methanone;
[6-chloro-3-(1H-1,2,3,4-tetazol-5-ylmethyl)-1H-indol-2-yl](1-methyl-2-imidazolyl)-methanone;
[6-chloro-3-(1H-1,2,3,4-tetrazol-5-ylmethyl)-1H-indol-2-yl](3-isoquinolinyl)-methanone;
[6-chloro-3-(1H-1,2,3,4-tetrazol-5-ylmethyl)-1H-indol-2-yl](2-quinolinyl)methanone;
[5-chloro-3-(1H-1,2,3,4-tetrazol-5-ylmethyl)-1H-indol-2-yl](4-methyl-2-pyridinyl)-methanone;
[6-trifluoromethyl-3-(1H-1,2,3,4-tetrazol-5-ylmethyl)-1H-indol-2-yl](4-methyl-2-pyridinyl)methanone;
[6-trifluoromethyl-3-(1H-1,2,3,4-tetrazol-5-ylmethyl)-1H-indol-2-yl](3-isoquinolinyl) methanone;
[5-trifluoromethyl-3-(1H-1,2,3,4-tetrazol-5-ylmethyl)-1H-indol-2-yl](4-methyl-2-pyridinyl)methanone;
[5-fluoro-3-(1H-1,2,3,4-tetrazol-5-ylmethyl)-1H-indol-2-yl](4-methyl-2-pyridinyl)-methanone;
[6-fluoro-3-(1H-1,2,3,4-tetrazol-5-ylmethyl)-1H-indol-2-yl](4-methyl-2-pyridinyl)-methanone;
[6-methyl-3-(1H-1,2,3,4-tetrazol-5-ylmethyl)-1H-indol-2-yl](4-methyl-2-pyridinyl)-methanone; and the salts thereof.

Most preferred individual compounds of this invention are:
[6-chloro-3-(1H-1,2,3,4-tetrazol-5-ylmethyl)-1H-indol-2-yl](4-methyl-2-pyridinyl)-methanone;
{6-chloro-3-[(2-methyl-2H-1,2,3,4-tetrazol-5-yl)methyl]-1H-indol-2-yl}(4-methyl-2-pyridinyl)methanone;
6-chloro-3-[(1-methyl-1H-1,2,3,4-tetrazol-5-yl)methyl]-1H-indol-2-yl}(4-methyl-2-pyridinyl)methanone;
[6-chloro-3-(1H-1,2,3,4-tetrazol-5-ylmethyl)-1H-indol-2-yl](5-methyl-2-pyridinyl)-methanone;
[6-chloro-3-(1H-1,2,3,4-tetrazol-5-ylmethyl)-1H-indol-2-yl](4-ethyl-2-pyridinyl)-methanone;
[6-chloro-3-(1H-1,2,3,4-tetrazol-5-ylmethyl)-1H-indol-2-yl](4-chloro-2-pyridinyl)-methanone;
[6-chloro-3-(1H-1,2,3,4-tetrazol-5-ylmethyl)-1H-indol-2-yl](5-chloro-2-pyridinyl)-methanone;
[6-chloro-3-(1H-1,2,3,4-tetrazol-5-ylmethyl)-1H-indol-2-yl](4-methoxy-2-pyridinyl)-methanone;
[6-chloro-3-(1H-1,2,3,4-tetrazol-5-ylmethyl)-1H-indol-2-yl](5-methyl-2-thienyl)-methanone;
{6-chloro-3-[(1-methyl-1H-1,2,3,4-tetrazol-5-yl)methyl]-1H-indol-2-yl}(5-methyl-2-thienyl)methanone;
{6-chloro-3-[(2-methyl-2H-1,2,3,4-tetrazol-5-yl)methyl]-1H-indol-2-yl}(5-methyl-2-thienyl)methanone;
[6-chloro-3-(1H-1,2,3,4-tetrazol-5-ylmethyl)-1H-indol-2-yl](3-isoquinolinyl)-methanone;
[6-trifluoromethyl-3-(1H-1,2,3,4-tetrazol-5-ylmethyl)-1H-indol-2-yl](3-isoquinolinyl) methanone;
[5-trifluoromethyl-3-(1H-1,2,3,4-tetrazol-5-ylmethyl)-1H-indol-2-yl](4-methyl-2-pyridinyl)methanone;
[5-fluoro-3-(1H-1,2,3,4-tetrazol-5-ylmethyl)-1H-indol-2-yl](4-methyl-2-pyridinyl)-methanone;
[6-fluoro-3-(1H-1,2,3,4-tetrazol-5-ylmethyl)-1H-indol-2-yl](4-methyl-2-pyridinyl)-methanone; and the salts thereof.

### General Synthesis

A compound of general formula (I) may be prepared by any synthetic procedure applicable to structure-related compounds known to those skilled in the art.

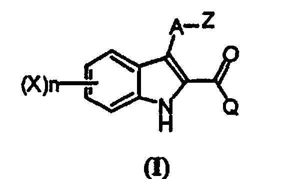

The following representative examples as described hereinafter are illustrative and are not meant to limit the scope of the invention in anyway. Unless otherwise stated, A, Q, X, Z, and n are as defined herein before.

### Scheme 1:

Compounds of formula (I) wherein -A-Z is -CH(R¹)-tetrazolyl (R¹ = hydrogen, C₁₋₄ alkyl or halosubstituted C₁₋₄ alkyl) may be prepared as indicated below.

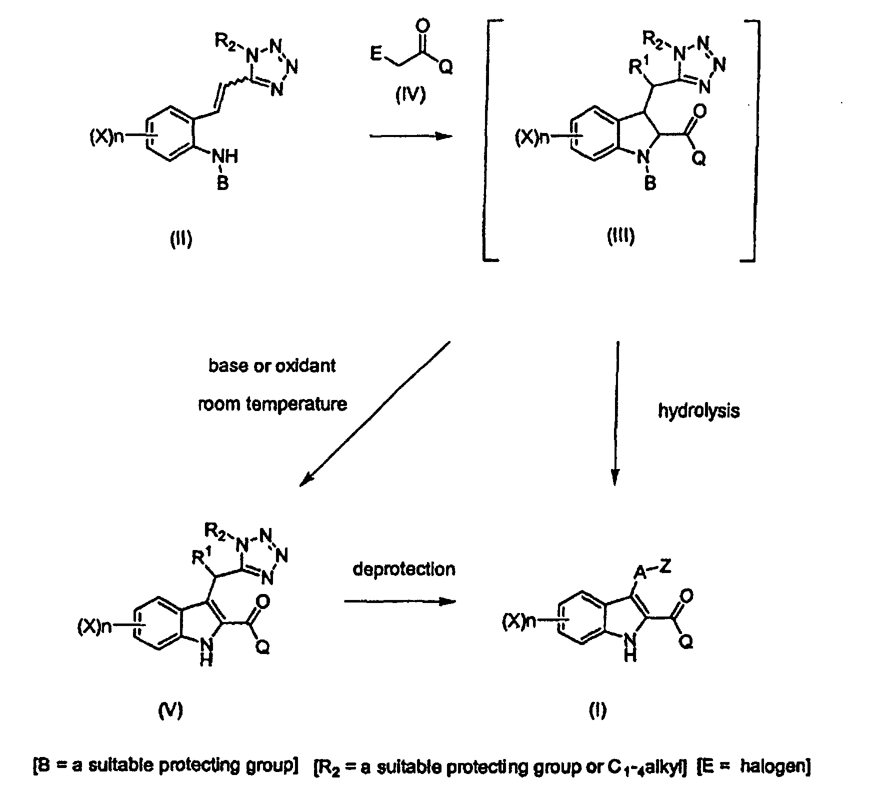

In the above reaction scheme, a compound of formula (I) is readily accessible from the appropriate 2-aminocinnamic tetrazole (II) wherein R¹ is hydrogen or C₁₋₄ alkyl; R² is a suitable protecting group or C₁₋₄ alkyl; and B is a suitable protecting group. Suitable protecting groups include, for example, methoxycarbonyl, ethoxycarbonyl, tert-butoxycarbonyl (Boc), benzyloxycarbonyl, phenylsulfonyl, p-toluenesulfonyl, methanesulfonyl and the like. The requisite 2-aminocinnamic tetrazole (II) is reacted with a compound of formula (IV) wherein E is halogen (preferably, iodo, bromo or chloro) in the presence of a suitable base. Suitable bases include, for example, an alkali or alkaline earth metal alkoxide, carbonate, or hydride, such as sodium *tert*-butoxide, potassium *tert*-butoxide, sodium carbonate, potassium carbonate, cesium carbonate, sodium hydride or potassium hydride. Preferred reaction inert solvents include, but are not limited to, acetone, methyl ethyl ketone, acetonitrile, N,N-dimethylformamide, N,N-dimethylacetamide, dimethylsulfoxide, dioxane and tetrahydrofuran. Reaction temperatures are preferably in the *range* of -40 °C to reflux temperature of the solvent, usually in the range of 0 °C to 60 °C, but if necessary, lower or higher temperature can be employed. Reaction time is in general from several minutes to a day, preferably from 30 minutes to 8 hours, however shorter or longer reaction times, if necessary, can be employed. When the reaction is, for example, conducted at room temperature the intermediate indoline (III) can be isolated. Reaction at higher temperatures can result in formation of indole (V).

Usually the intermediate indoline (III) is not isolated but either (i) hydrolyzed with concomitant formation of the indole ring directly to a compound of formula (I) under standard conditions known to those skilled in the art, or (ii) transformed to a compound of formula (V) by using a suitable base or a suitable oxidant. Suitable bases include, for example, an alkali or alkaline earth metal carbonate such as sodium carbonate, potassium carbonate or cesium carbonate, or an organic base such as 1,8-diazabicyclo[5.4.0]undec-7-ene (DBU), 1,5-diazabicyclo[4.3.0]non-5-ene (DBN), 1,4-diazabicyclo[2.2.2]octane (DABCO), pyridine, pyrrolidine, triethylamine, diethylisopropylamine or Hunig's base. Suitalbe oxidants include cerium(IV) ammonium nitrate (CAN), manganese(IV) oxide, manganese(III) triacetate, copper(II) acetate / air, chloranil, 2,3-dichloro-5,6-dicyano-1,4-benzoquinone (DDQ), N-methylmorpholine N-oxide and the like (for example, see H. Dumoulin et al; J. Heterocycl. Chem., **32**, 1703, 1995; H. Rapoport et al; Tetrahedron Lett., 5053, 1991; P. Martin et al; Helv. Chim. Acta, **77,** 111, 1994; Y. Kikugawa et al, J. Chem. Soc. Perkins Trans 1, **7**, 1401, 1984; A. Goti et al; Tetrahedron Lett., 6567, 1996; L. S. Liebeskind et al; J. Org. Chem, **61,** 2594, 1996). Preferred reaction inert solvents include acetone, methyl ethyl ketone, acetonitrile, dioxane or tetrahydrofuran. Reaction temperatures are preferably in the range of 0 °C to reflux temperature of solvent, usually in the range of 15 to 60 °C, but if necessary, lower or higher temperature can be employed. Reaction time is in general from one minute to 24 hours, preferably from 30 minutes to 8 hours, however shorter or longer reaction times, if necessary, can be employed. When the group of R² is a suitable protecting group, a compound of formula (V) may be readily converted to a compound of formula (I) under a number of standard procedures known to those skilled in the art (for example, see "Protection of the Amino Group', in Protective Groups in Organic Synthesis, 2nd Edition, T.W.Greene and P.G.M.Wuts, Ed., John Wiley and Sons, Inc.1991, pp.309-405.; John V.Duncia, Michael E.Pierce, and Joseph B.Santella III, J.Org.Chem., **56**, 2395, 1991).

### Scheme 2:

Compounds of formula (Ia) (e.g., a compound of formula (Ia) wherein Z is non-substituted tetrazolyl) may be prepared from a compound of formula (VII) by a number of synthetic procedures known to those skilled in the art (for example, see Comprehensive Heterocyclic Chemistry Vol. 5, Kelvin T.Potts, Ed., Pergamon Press, Oxford, 1984, pp.791-838.; Comprehensive Heterocyclic Chemistry II Vol. 4, Richard C.Storr, Ed., Elsevier Science, Oxford, 1996, pp.612-678).

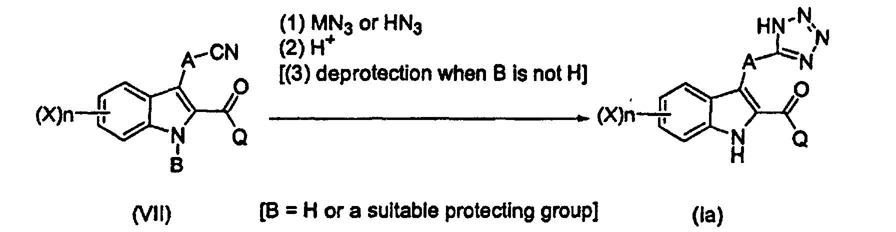

For example, as depicted in the above reaction scheme, a compound of formula (VII) wherein B is hydrogen or a suitable protecting group, is treated with HN₃ or a compound of formula MN₃ in a reaction inert solvent. Suitable protecting groups (as B) include, for example, methoxycarbonyl, ethoxycarbonyl, tert-butoxycarbonyl (Boc), benzyloxycarbonyl, phenylsulfonyl, p-toluenesulfonyl and methanesulfonyl. MN₃ may be selected from azidotrimethyltin (Me₃SnN₃), azidotributyltin ((n-Bu)₃SnN₃), sodium azide (NaN3), lithium azide (LiN₃), ammonium azide (NH₄N₃) and azidotrimethylsilane (Me₃SiN₃). When trimethylsilyl azide (Me₃SiN₃) is used as an azide source, preferably, the instant reaction is conducted in the presence of a suitable Lewis acid. Suitable Lewis acids include trimethyl aluminum (Me₃Al), tin(II) chloride (SnCl₂) and tin(IV) chloride (SnCl₄). (for example, S.Kirchmeyer et al., Synthesis, 500, 1983.; K.Nishiyama et al., Synthesis, 106, 1988; B.E.Huff et al., Tetrahedron Lett, 8011, 1993). Preferred reaction inert solvents include, but are not limited to, N,N-dimethylformamide, N,N-dimethylacetamide, dimethylsulfoxide, acetone, methyl ethyl ketone, dioxane, tetrahydrofuran, nitromethane, toluene and xylene. Reaction temperatures are preferably in the range of -40 °C to reflux temperature of the solvent, usually in the range of 0 °C to reflux temperature of the solvent, but if necessary, lower or higher temperatures can be employed. Reaction time is in general from one minute to 24 hours, preferably from 30 minutes to 8 hours, however shorter or longer reaction times, if necessary, can be employed. The work-up procedures involve the freeing of the anionic tetrazole salt by acid treatment. When the group B is a suitable protecting group as defined herein above, the group B may be cleaved by a number of standard procedures known to those skilled in the art (for example, see "Protection of the Amino Group', in Protective Groups in Organic Synthesis, 2nd Edition, T.W.Greene and P.G.M.Wuts, Ed., John Wiley and Sons, Inc.1991, pp.309-405).

### Scheme 3:

Compounds of formula (Ib) (e.g., a compound of formula (I) wherein Z is substituted tetrazolyl) may be prepared from a compound of formula (IX) by a number of synthetic procedures known to those skilled in the art (for example, see Comprehensive Heterocyclic Chemistry Vol. 5, Kelvin T.Potts, Ed., Pergamon Press, Oxford, 1984, pp.791-838., Comprehensive Heterocyclic Chemistry II Vol. 4, Richard C.Storr, Ed., Elsevier Science, Oxford, 1996, pp.612-678).

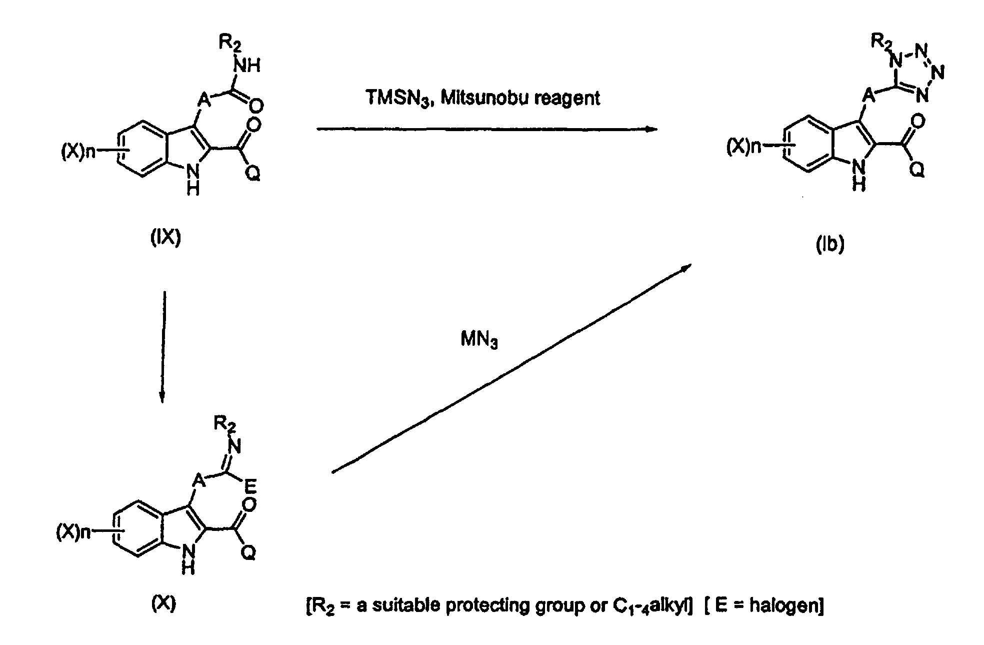

For example, as illustrated in the above reaction scheme, a compound of formula (IX) wherein R² is a suitable protecting group, C₁₋₄ alkyl or halosubstituted C₁₋₄ alkyl, is reacted with trimethylsilyl azide usually under Mitsunobu reaction conditions to afford the corresponding tetrazole compound (Ib). (for example, see John V.Duncia et al., J.Org.Chem., **56**, 2395, 1991).

Altenatively, a compound of formula (Ib) may be obtained from a compound of formula (X) wherein E is halogen (preferably, iodo, bromo or chloro). For example, a compound of formula (X) is treated with a compound of formula MN₃ as defined before, in a reaction inert solvent. Preferred reaction inert solvents include, but are not limited to, N,N-dimethylformamide, N,N-dimethylacetamide, dimethylsulfoxide, acetone, methyl ethyl ketone, dioxane, tetrahydrofuran, nitromethane, toluene and xylene. Reaction temperatures are preferably in the range of -40 °C to reflux temperature of solvent, usually in the range of 0 °C to reflux temperature of the solvent, but if necessary, lower or higher temperatures can be employed. Reaction time is in general from one minute to 24 hours, preferably from 30 minutes to 8 hours, however shorter or longer reaction times, if necessary, can be employed. A compound of formula (X) may be prepared from a compound of formula (IX) by using, for example, phosphorus pentachloride (PCl5), phosphorus oxychloride (POCl3) or triphenyl phosphine (Ph₃P)/CCl₄.

When the group of R² is a suitable protecting group, a compound of formula (Ib) may be readily converted to a compound of formula (Ia) under standard procedures known to those skilled in the art (for example, see "Protection of the Amino Group', in Protective Groups in Organic Synthesis, 2nd Edition, T.W.Greene and P.G.M.Wuts, Ed., John Wiley and Sons, Inc.1991, pp.309-405., John V.Duncia, Michael E.Pierce, and Joseph B.Santella III, J.Org.Chem., **56,** 2395, 1991).

### Scheme 4:

In another embodiment, compounds of formula (Ib) wherein R² is C₁₋₄ alkyl or halosubstituted C₁₋₄ alkyl, and a compound of formula (Ic) wherein R² is as defined above, may be prepared from a compound (Ia) as illustrated bellow.

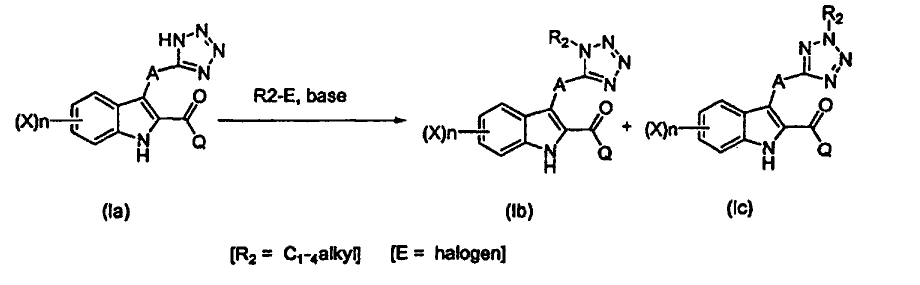

For example, a compound of formula (Ia) is reacted with a compound of formula R²-E wherein E is halogen (preferably, iodo, bromo or chloro) in the presence of a suitable base. Suitable bases include, for example, an alkali or alkaline earth metal alkoxide, carbonate, or hydride, such as sodium *tert*-butoxide, potassium *tert*-butoxide, sodium carbonate, potassium carbonate, cesium carbonate, sodium hydride or potassium hydride. Preferred reaction inert solvents include, but are not limited to, acetone, methyl ethyl ketone, acetonitrile, N,N-dimethylformamide, N,N-dimethylacetamide, dimethylsulfoxide, dioxane or tetrahydrofuran. Reaction temperatures are preferably in the range of -40 °C to reflux temperature of solvent, usually in the range of 0 °C to 60 °C, but if necessary, lower or higher temperature can be employed. Reaction time is in general from several minutes to a day, preferably from 30 minutes to 8 hours, however shorter or longer reaction times, if necessary, can be employed.

### Scheme 4:

Compounds of formula (II) may be prepared by a number of synthetic procedures known to those skilled in the art, for example by the following method..

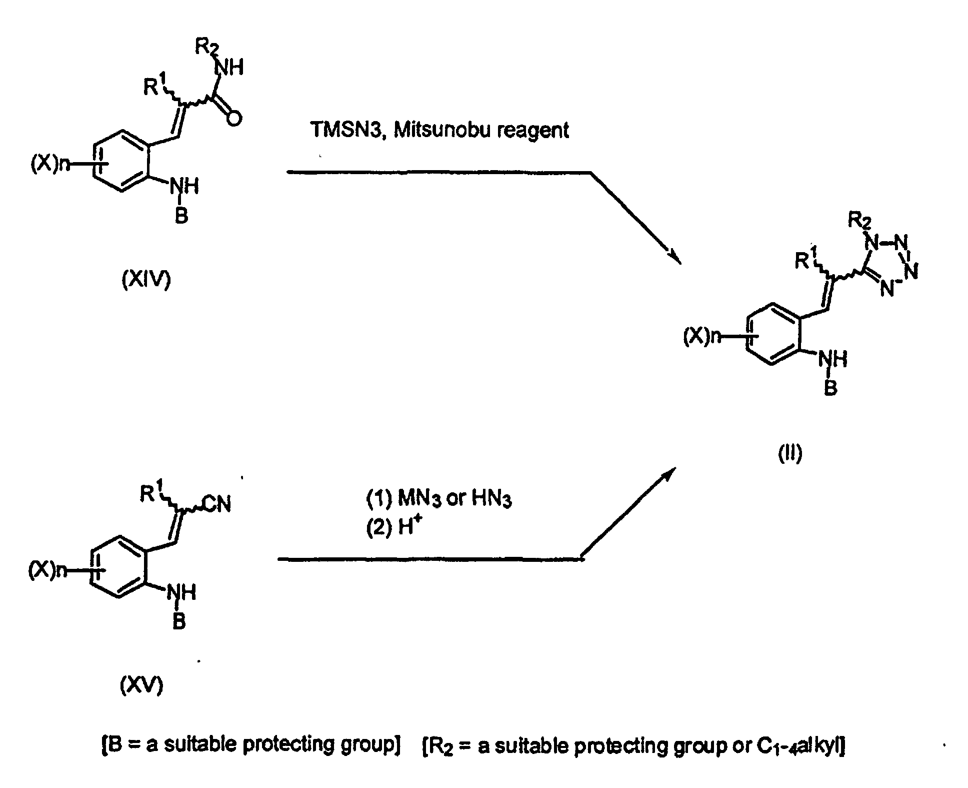

For example, as depicted in the above reaction shceme, a compound (II) may be prepared by treatment of a compound of formula (XIV), with trimethylsilyl azide usually under Mitsunobu reaction conditions to afford the corresponding tetrazole compound (II). (for example, see John V.Duncia et al., J.Org.Chem., **56**, 2395, 1991).

Altenatively, a compound (II) may be obtained from a compound of formula (XV). For example, a compound (XV) is treated with HN₃ or a compound of formula MN₃ in a reaction inert solvent. MN₃ may be selected from azidotrimethyltin (Me₃SnN₃), azidotributyltin ((n-Bu)₃SnN₃), sodium azide (NaN₃), lithium azide (LiN3), ammonium azide (NH₄N₃) and azidotrimethylsilane (Me₃SiN₃). When trimethylsilyl azide (Me₃SiN₃) is used as an azide source, preferably, the instant reaction is conducted in the presence of a suitable Lewis acid. Suitable Lewis acids include trimethyl aluminum (Me₃Al), tin(II) chloride (SnCl₂), tin(IV) chloride (SnCl₄). (for example, S.Kirchmeyer et al., Synthesis, 500, 1983.; K.Nishiyama et al., Synthesis, 106, 1988; B.E.Huff et al., Tetrahedron Lett, 8011, 1993). Preferred reaction inert solvents include, but are not limited to, N,N-dimethylformamide, N,N-dimethylacetamide, dimethylsulfoxide, acetone, methyl ethyl ketone, dioxane, tetrahydrofuran, nitromethane, toluene or xylene. Reaction temperatures are preferably in the range of -40 °C to reflux temperature of solvent, usually in the range of 0 °C to reflux temperature of solvent, but if necessary, lower or higher temperatures can be employed. Reaction, time is in general from several minutes to a day, preferably from 30 minutes to 8 hours, however shorter or longer reaction times, if necessary, can be employed.

The starting materials in the aforementioned general syntheses may be obtained by conventional methods known to those skilled in the art. The preparation of such starting materials is described within the accompanying non-limiting examples which are provided for the purpose of illustration only. Alternatively, requisite starting materials may be obtained by analogous procedures, or modifications thereof, to those described hereinafter.

The products which are addressed in the aforementioned general syntheses and illustrated in the experimental examples described herein after may be isolated by standard methods and purification can be achieved by conventional means known to those skilled in the art, such as distillation, crystallization or chromatography techniques.

Certain compounds described herein contain one or more asymmetric centers and are capable of existing in various stereoisomeric forms. The present invention contemplates all such possible stereoisomers as well as their racemic and resolved, enantiomerically pure forms and pharmaceutically acceptable salts thereof.

Certain compounds of the present invention are capable of forming addition salts with inorganic or organic acids. The pharmaceutically acceptable acid salts of the compounds of formula (I) are those which form non-toxic addition salts, such as, but not limited to, the hydrochloride, hydrobromide, sulfate or bisulfate, acetate, benzoate, besylate, citrate, fumarate, glucuronate, hippurate, lactate, tartrate, saccharate, succinate, maleate, methanesulfonate, *p*-toluenesulfonate, phosphate and pamoate (i.e., 4,4'-methylene-*bis*-(3-hydroxy-2-naphthoate)) salts. The pharmaceutically acceptable acid salts may be prepared by conventional techniques.

Certain compounds of the present invention are capable of forming pharmaceutically acceptable non-toxic cations. Pharmaceutically acceptable non-toxic cations of compounds of formula (I) may be prepared by conventional techniques by, for example, contacting said compound with a stoichiometric amount of an appropriate alkali or alkaline earth metal (sodium, potassium, calcium and magnesium) hydroxide or alkoxide in water or an appropriate organic solvent such as ethanol, isopropanol, mixtures thereof, or the like.

Also included within the scope of this invention are bioprecursors (also called pro-drugs) of the compounds of the formula (I). A bioprecursor of a compound of the formula (I) is a chemical derivative thereof which is readily converted back into the parent compound of the formula (I) in biological systems. In particular, a bioprecursor of a compound of the formula (I) is converted back to the parent compound of the formula (I) after the bioprecursor has been administered to, and absorbed by, a mammalian subject, e.g., a human subject. When the compounds of the formula (I) of this invention may form solvates such as hydrates, such solvates are included within the scope of this invention.

Examples of prodrug of the compounds of the formula (I) are those compounds of the formula (I) wherein the 1st position of indole ring is substituted with a group selected from hydroxymethyl, -C(O)-C₁₋₄ alkyl, -C(O)-(NH₂)CH-(C₁₋₄ alkyl), -C(O)-phenyl, -CH₂NHC(O)-aryl, -CH₂-C₁₋₄alkyl-O-C(O)-C₁₋₄alkyl, -C₁₋₄ alkyl-pyridyl, -C(O)CH₂NR₂ and -CH₂N(C₁₋₄ alkyl)₂.

The other examples of the prodrugs are those compounds of the formula (I) wherein the carboxyl group is substituted with a group selected from C₁₋₄ alkyl, -CH₂-C₁₋₄alkyl-O-C(O)-C₁₋₄alkyl, -CH₂-C₁₋₄alkyl-O-C(O)-N(C₁₋₄alkyl)₂, -CH₂C(O)-N(C₁₋₄ alkyl)₂, -CH₂-C₁₋₄alkyl-O-C(O)-O-C₁₋₄alkyl, ethyl-OH and -CH₂CO₂H.

The compounds of the formula (I) of this invention can be administered via either the oral, parenteral or topical routes to mammals. In general, these compounds are most desirably administered to humans in doses ranging from 0.01 mg to 100 mg per kg of body weight per day, although variations will necessarily occur depending upon the weight, sex and condition of the subject being treated, the disease state being treated and the particular route of administration chosen. However, a dosage level that is in the range of from 0.01 mg to 10 mg per kg of body weight per day, single or divided dosage is most desirably employed in humans for the treatment of abovementioned diseases.

The compounds of the present invention may be administered alone or in combination with pharmaceutically acceptable carriers or diluents by either of the above routes previously indicated, and such administration can be carried out in single or multiple doses. More particularly, the novel therapeutic agents of the invention can be administered in a wide variety of different dosage forms, i.e., they may be combined with various pharmaceutically acceptable inert carriers in the form of tablets, capsules, lozenges, trochees, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various nontoxic organic solvents, etc. Moreover, oral pharmaceutical compositions can be suitably sweetened and/or flavored. In general, the therapeutically-effective compounds of this invention are present in such dosage forms at concentration levels ranging 5% to 70% by weight, preferably 10% to 50% by weight.

For oral administration, tablets containing various excipients such as microcrystalline cellulose, sodium citrate, calcium carbonate, dipotassium phosphate and glycine may be employed along with various disintegrants such as starch and preferably corn, potato or tapioca starch, alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatine capsules; preferred materials in this connection also include lactose or milk sugar as well as high molecular weight polyethylene grycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various combinations thereof.

For parenteral administration, solutions of a compound of the present invention in either sesame or peanut oil or in aqueous propylene glycol may be employed. The aqueous solutions should be suitably buffered (preferably pH>8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well-known to those skilled in the art. Additionally, it is also possible to administer the compounds of the present invention topically when treating inflammatory conditions of the skin and this may preferably be done by way of creams, jellies, gels, pastes, ointments and the like, in accordance with standard pharmaceutical practice.

The compounds of formula (I) may also be administered in the form of suppositories for rectal or vaginal administration of the active ingredient. These compositions can be prepared by mixing the active ingredient with a suitable nonirritating excipient which is solid at room temperature (for example, 10 °C to 32 °C) but liquid at the rectal temperature and will melt in the rectum or vagina to release the active ingredient. Such materials are polyethylene glycols, cocoa butter, suppository and wax.

For buccal administration, the composition may take the form of tablets or lozenges formulated in conventional manner.

### Combination with Other Drugs:

Compounds of Formula I would be useful for, but not limited to, the treatment of inflammation in a subject, and for treatment of other inflammation-associated disorders, such as, as an analgesic in the treatment of pain and headaches, or as an antipyretic for the treatment of fever. For example, combinations of the invention would be useful to treat arthritis, including but not limited to rheumatoid arthritis, spondyloarthopathies, gouty arthritis, osteoarthritis, systemic lupus erythematosus and juvenile arthritis. Such combinations of the invention would be useful in the treatment of asthma, bronchitis, inmenstrual cramps, tendinitis, bursitis, and skin related conditions such as psoriasis, eczema, burns and dermatitis. Combinations of the invention also would be useful to treat gastrointestinal conditions such as inflammatory bowel disease. Crohn's disease, gastritis, irritable bowel syndrome and ulcerative colitis and for the prevention of colorectal cancer. Combinations of the invention would be useful in creating inflammation in such diseases as vascular diseases, migraine headaches, periarteritis nodosa, thyroiditis, aplastic anemia, Hodgkin's disease, sclerodoma, rheumatic fever, type I diabetes, myasthenia gravis, multiple sclercsis, sarcoidosis, nephrotic syndrome, Behcet's syndrome, polymyositis, gingivitis, hypersensitivity, Conjunctivitis, swelling occurring after injury, myocardial ischemia, and the like. The combinations would also be useful for the treatment of certain central nervous system disorders such as Alzheimer's disease and dimentia. The combinations of the invention are useful as anti-inflammatory agents, such as for the treatment of arthritis, with the additional benefit of having significantly less harmful side effects. These compositions would also be useful in the treatment of allergic rhinitis, respiratory distress syndrome, endotoxin shock syndrome, atherosclerosis and central nervous system damage resulting from stroke, ischemia and trauma.

Compounds of formula (I) will be useful as a partial or complete substitute for conventional NSAID's in preparations wherein they are presently co-administered with other agents or ingredients. Thus, the invention encompasses pharmaceutical compositions for treating COX-2 mediated diseases as defined above comprising a non-toxic therapeutically effective amount of the compound of formula (I) and one or more ingredients such as another pain reliever including acetaminophen or phenacetin; a potentiator including caffeine; an H₂-antagonist, aluminom or magnesium hydroxide, simethicone, a decongestant including phenylephrine, phenylproanolamine, psuedophedrine, oxymetazoline, ephinephrine, naphazoline, xylometazoline, propylhexedrine, or levodesoxyephedrine; an antiitussive including codeine, hydrocodone, caramiphen, carbetapentane, or dextramethorphan; a prostaglandin including misoprostol, enprostil, rioprostil, ornoprotol or rosaprostol; a diuretic; a sedating or non-sedating antihistamine; anticancer agents such as angiostatin and endostatin; anti-Alzheimers such as Doepezil and Tacrine hydrochloride; and TNF alpha inhibitors such as Etanercept.

These cyclooxygenase inhibitors can further be used in combination with a nitric oxide inhibitors disclosed in WO 96/28145.

Also, the invention encompasses pharmaceutical compositions for treating COX-2 mediated diseases as defined above comprising a non-toxic therapeutically effective amount of the compound of formula (I) and one or more anti-ulcer agent and/or prostaglandins, which are disclosed in WO 97/11701.

The useful prostaglandins include misoprostol, plus-minus methyl 11α, 16-dihydroxy-16-methyl-9-oxoprost 13E-en-1-oate; enisoprost and methyl-7-[2B-[6-(1-cyclopenten-1-yl)-4-hydroxy-4-methyl-lE, 5E-hexadienyl]-3α-hydroxy-5-oxo 1R, 1α-cyclopentyl]-4Z-heptenoate. Prostaglandins within the scope of the invention also include arbaprostil, enprostil, rioprostol, nocloprost, mexiprostil, ornoprostol, dimoxaprost, tiprostanide and rosaprostol.

The present compounds may also be used in co-therapies, partially or completely, in place of other conventional antiinflammatories, such as together with steroids, 5-lipoxygenase inhibitors, LTB₄ antagonists and LTA₄ hydrolase inhibitor's.

An example of LTB₄ is disclosed in WO97/29774. Suitable LTB₄ inhibitors include, among others, ebselen, Bayer Bay-x-1005, Ciba Geigy compound CGS-25019C, Leo Denmark compound ETH-615, Lilly compound LY-293111, Ono compound ONO-4057, Terumo compound TMK-688, Lilly compounds LY-213024, 264086 and 292728, Ono compound ONO-LB457, Searle compound SC-S3228, calcitrol, Lilly compounds LY-210073, LY223982, LY233469, and LY255283, Ono compound ONO-LB-448, Searle compounds SC-41930, SC-50605 and SC-51146, and SK&F compound SKF-104493. Preferably, the LTB₄ inhibitors are selected from ebselen, Bayer Bay-x-1005, Ciba Geigy compound CGS-25019C, Leo Denmark compound ETH-61S, Lilly compound LY-293111, Ono compound ONO-4057 and Terumo compound TMK-688.

An example of 5-LO inhibitors is disclosed in WO97/29776. Suitable 5-LO inhibitors include, among others, masoprocol, tenidap, zileuton, pranlukast, tepoxalin, rilopirox, flezelastine hydrochloride, enazadrem phosphate and bunaprolast.

An example of LTA₄ hydrolase inhibitors is disclosed in WO97/29774. Suitable LTA₄ hydrolase inhibitors include, among others, Rhone-Poulenc Rorer RP-64966.

The administration of the present invention may be for either prevention or treatment purposes. The methods and compositions used herein may be used alone or in conjunction with additional therapies known to those skilled in the art in the prevention or treatment of angiogenesis. Alternatively, the methods and compositions described herein may be used as adjunct therapy. By way of example, the cyclooxygenase-2 inhibitor may be administered alone or in conjunction with other antineoplastic agents or other growth inhibiting agents or other drugs or nutrients.

There are large numbers of antineoplastic agents available in commercial use, in clinical evaluation and in pre-clinical development, which could be selected for treatment of angiogenesis by combination drug chemotherapy. Such antineoplastic agents fall into several major categories, namely, antibiotic-type agents, alkylating agents, antimetabolite agents, hormonal agents, immunological agents, interferon-type agents and a category of miscellaneous agents. Alternatively, other anti-neoplalstic agents , such as metallomatrix proteases inhibitors (MMP), such as MMP-13 inhibitors including batiastat, marimastat. Agouron Pharmaceuticals AG-3340, and Roche R0-32-3555, or alpha,beta,inhibitors may be used.

A first family of antineoplastic agents which may be used in combination with a selective cyclooxygenase-2 inhibitor consists of antimetabolite-type antineoplastic agents. Suitable antimetabolite antineoplastic agents may be selected from the group consisting of 5-FU-fibrinogen, acanthifolic acid, aminothiadiazole, brequinar sodium, carmofur, Ciba-Geigy CGP-30694, cyclopentyl cytosine, cytarabine phosphate stearate, cytarabine conjugates, Lilly DATHF, Merrel Dow DDFC, dezaguanine, dideoxycytidine, dideoxyguanosine, didox, Yoshitomi DMDC, doxifluridine, Wellcome EHNA, Merck & Co. EX-015, fazarabine, floxuridine, fludarabine phosphate, 5-fluorouracil, N-(2'-furanidyl)-5-fluorouracil, Daiichi Seiyaku F0-152, isopropyl pyrrolizine, Lilly LY-188011, Lilly LY-264618, methobenzaprim, methotrexate, Wellcome MZPES. norspermidine, NCI NSC-127716, NCI NSC-264880, NCI NSC-39661, NCI NSC-612567, Warner-Lambert PALA, pentostatin, piritrexim, plicamycin, Asahi Chemical PL-AC, Takeda TAC-788, thioguanine, tiazofurin, Erbamont TIF, trimetrexate, tyrosine kinase inhibitors, tyrosine protein kinase inhibitors, Taiho UFT and uricytin.

A second family of antineoplastic agents which may be used in combination with a selective cyclooxygenase-2 inhibitor consists of alkylating-type antineoplastic agents. Suitable alkylating-type antineoplastic agents may be selected from the group consisting of Shionogi 254-S, aldo-phosphamide analogues, altretamine, anaxirone, Boehringer Mannheim BBR-2207, bestrabucil, budotitane, Wakunaga CA-102, carboplatin, carmustine, Chinoin-139, Chinoin-153, chlorambucil, cisplatin, cyclophosphamide, American Cyanamid CL-286558, Sanofi CY-233, cyplatate, Degussa D-19-384, Sumimoto DACHP(Myr)2, diphenylspiromustine, diplatinum cytostatic. Erba distamycin derivatives, Chugai DWA-2114R, ITI E09, elmustine, Erbamont FCE-24517, estramustine phosphate sodium, fotemustine, Unimed G-6-M, Chinoin GYKI-17230, hepsul-fam, ifosfamide, iproplatin, Iomustine, mafosfamide, mitolactol, Nippon Kayaku NK-121, NCI NSC-264395, NCI NSC-342215, oxaliplatin, Upjohn PCNU, prednimustine, Proter PTT-119, ranimustine, semustine, SmithKline SK&F-l01772, Yakult Honsha SN-22, spiromus-tine, Tanabe Seiyaku TA-077, tauromustine, temozolomide, teroxirone, tetraplatin and trimelamol.

A third family of antineoplastic agents which may be used in combination with a selective cyclooxygenase-2 inhibitor consists of antibiotic-type antineoplastic agents. Suitable antibiotic-type antineoplastic agents may be selected from the group consisting of Taiho 4181-A, aclarubicin, actinomycin D, actinoplanone, Erbamont ADR-456, aeroplysinin derivative, Ajinomoto AN-201-II. Ajinomoto AN-3, Nippon Soda anisomycins, anthracycline, azino-mycin-A, bisucaberin, Bristol-Myers BL-6859, Bristol-Myers BMY-25067. Bristol-Myers BMY-25551, Bristol-Myers BMY-26605, Bristol-Myers BMY-27557, Bristol-Myers BMY-28438, bleomycin sulfate, bryostatin-1, Taiho C-l027, calichemycin, chromoximycin, dactinomycin, daunorubicin, Kyowa Hakko DC-102, Kyowa Hakko DC-79, Kyowa Hakko DC-88A, Kyowa Hakko DC89-Al, Kyowa Hakko DC92-B, ditrisarubicin B, Shionogi DOB-41, doxorubicin, doxorubicin-fibrinogen, elsamicin-A, epirubicin, erbstatin, esorubicin, esperamicin-A1, esperamicin-Alb. Erbamont FCE-21954, Fujisawa FK-973, fostriecin, Fujisawa FR-900482, glidobactin, gregatin-A, grincamycin, herbimycin, idarubicin, illudins, kazusamycin, kesarirhodins, Kyowa Hakko KM-5539, Kirin Brewery KRN-8602, Kyowa Hakko KT-5432, Kyowa Hakko KT-5594, Kyowa Hakko KT-6149, American Cyanamid LL-D49194, Meiji Seika ME 2303, menogaril, mitomycin, mitoxantrone, SmithKline M-TAG, neoenactin, Nippon Kayaku NK-313, Nippon Kayaku NKT-Ol, SRI International NSC-357704, oxalysine, oxaunomycin, peplomycin, pilatin, pirarubicin, porothramycin, pyrindamycin A, Tobishi RA-I, rapamycin, rhizoxin, rodorubicin, sibanomicin, siwenmycin, Sumitomo SM-5887, Snow Brand SN-706, Snow Brand SN-07, sorangicin-A, sparsomycin, SS Pharmaceutical SS-21020, SS Pharmaceutical SS-7313B, SS Pharmaceutical SS-9816B, steffimycin B, Taiho 4181-2, talisomycin, Takeda TAN-868A, terpentecin, thrazine, tricrozarin A, Upjohn U-73975, Kyowa Hakko UCN-10028A, Fujisawa WF-3405, Yoshitomi Y-2S024 and zorubicin.

A fourth family of antineoplastic agents which may be used in combination with the selective cyclooxygenase-2 inhibitor consists of a miscellaneous family of antineoplastic agents selected from the group consisting of alpha-carotene, alpha-difluoromethyl-arginine, acitretin, Biotec AD-5, Kyorin AHC-52, alstonine, amonafide, amphethinile. amsacrine, Angiostat, ankinomycin, anti-neoplaston AIO, antineoplaston A2, antineoplaston A3, antineoplaston A5, antineoplaston AS2-1, Henkel APD, aphidicolin glycinate, asparaginase, Avarol, baccharin, batracylin, benfluron, benzotript, Ipsen-Beaufour BIM-23015, bisantrene, Bristo-Myers BMY-40481, Vestar boron-lO, bromofosfamide, Wellcome BW-502, Wellcome BW-773, caracemide, carmethizole hydrochloride, Ajinomoto CDAF, chlorsulfaquinoxalone, Chemes CHX-2053, Chemex CHX-1OO, Warner-Lambert CI-921, Warner-Lambert CI-937, Warner-Lambert CI-941, Warner-Lambert CI-958, clanfenur, claviridenone, ICN compound 1259, ICN compound 4711, Contracan, Yakult Honsha CPT-11, crisnatol, curaderm, cytochalasin B, cytarabine, cytocytin, Merz D-609, DABIS maleate, dacarbazine, datelliptinium, didemnin-B, dihaematoporphyrin ether, dihydrolenperone, dinaline, distamycin, Toyo Pharmar DM-341, Toyo Pharmar DM-75, Daiichi Seiyaku DN-9693, elliprabin, elliptinium acetate, Tsumura EPMTC, ergotamine, etoposide, etretinate, fenretinide, Fujisawa FR-57704, gallium nitrate, genkwadaphnin, Chugai GLA-43, Glaxo GR-63178, grifolan NMF-5N, hexadecylphosphocholine, Green Cross H0-221, homoharringtonine, hydroxyurea, BTG ICRF-187, ilmofosine, isoglutamine, isotretinoin. Otsuka JI-36, Ramot K-477, Otsuak K-76COONa, Kureha Chemical K-AM, MECT Corp KI-8110, American Cyanamid L-623, leukoregulin, Ionidamine, Lundbeck LU-23-112, Lilly LY-186641, NCI (US) MAP, marycin, Merrel Dow MDL-27048, Medco MEDR-340, merbarone, merocyanine derivatives, methylanilinoacridine, Molecular Genetics MGI-136, minactivin, mitonafide, mitoquidone, mopidamol, motretinide, Zenyaku Kogyo MST-16, N-(retinoyl)amino acids, Nisshin Flour Milling N-021, N-acylated-dehydroalanines, nafazatrom, Taisho NCU-190, nocodazole derivative, Normosang, NCI NSC-145813, NCI NSC-361456, NCI NSC-604782, NCI NSC-95580, octreotide, Ono ON0-112, oquizanocine, Akzo Org-l0172, pancratistatin, pazelliptine, Warner-Lambert PD-111707, Warner-Lambert PD-115934, Warner-Lambert PD-131141, Pierre Fabre PE-1OO1, ICRT peptide D, piroxantrone, polyhaematoporphyrin, polypreic acid, Efamol porphyrin, probimane, procarbazine, proglurnide, Invitron protease nexin I, Tobishi RA-700, razoxane, Sapporo Breweries RBS, restrictin-P, retelliptine, retinoic acid, Rhone-Poulenc RP-49532, Rhone-Poulenc RP-56976, SmithKline SK&F-l04864, Sumitomo SM-108, Kuraray SMANCS, SeaPharm SP-l0094, spatol, spirocyclopropane derivatives, spirogermanium, Unimed, SS Pharmaceutical SS-554, strypoldinone, Stypoldione, Suntory SUN 0237, Suntory SUN 2071, superoxide dismutase, Toyama T-506, Toyama T-680, taxol, Teijin TEI-0303, teniposide, thaliblastine, Eastman Kodak TJB-29, tocotrienol, Topostin, Teijin TT-82, kyowa Hakko UCN-Ol, Kyowa Hakko UCN-1028, ukrain, Eastman Kodak USB-006, vinblastine sulfate, vincristine, vindesine, vinestramide, vinorelbine, vintriptol, vinzolidine, withanolides and Yamanouchi YM-534.

Examples of radioprotective agents which may be used in the combination chemotherapy of this invention are AD-5, adchnon, amifostine analogues, detox, dimesna, 1-102, MN-159, N-acylated-dehydroalanines, TGF-Genentech, tiprotimod, amifostine, WR-151327, FUT-187, ketoprofen transdermal, naburnetone, superoxide dismutase (Chiron) and superoxide disrrtutase Enzon.

Methods for preparation of the antineoplastic agents described above may be found in the literature. Methods for preparation of doxorubicin, for example, are described in U.S. Patents No. 3,590,028 and No. 4,012,448. Methods for preparing metallomatrix protease inhibitors are described in EP 780386, W097/20824. W096/15096. Methods for preparing SOD mimics are described in EP 524,101. Methods for preparing alpha,beta, inhibitors are described in W097/08174.

In addition, the selective COX-2 inhibitor may be administered in conjunction with other antiinflammatory agents for maximum safety and efficacy, including NSAID's, selective COX-1 inhibitors and inhibitors of the leukotriene pathway, including 5-lipoxygenase inhibitors. Examples of NSAID's include indomethacin, naproxen, ibruprofen, salicylic acid derivatives such as aspirin, diclofenac, ketorolac, piroxicam, meloxicam, mefenamic acid, sulindac, tolmetin sodium, zomepirac, fenoprofen, phenylbutazone, oxyphenbutazone, nimesulide, zaltoprofen and letodolac.

### Method for assessing biological activities:

The activity of the compounds of the formula (I) of the present invention was demonstrated by the following assays.

### In vitro assays

### Human cell based COX-1 assay

Human peripheral blood obtained from healthy volunteers was diluted to 1/10 volume with 3.8% sodium citrate solution. The platelet-rich plasma immediately obtained was washed with 0.14 M sodium chloride containing 12 mM Tris-HCl (pH 7.4) and 1.2 mM EDTA. Platelets were then washed with platelet buffer (Hanks buffer (Ca free) containing 0.2% BSA and 20 mM Hepes). Finally, the human washed platelets (HWP) were suspended in platelet buffer at the concentration of 2.85 x 10⁸ cells/ml and stored at room temperature until use. The HWP suspension (70 µl aliquots, final 2.0 x 10⁷ cells/ml) was placed in a 96-well U bottom plate and 10 µl aliquots of 12.6 mM CaCl₂ added. Platelets were incubated with A23187 (final 10 µM, Sigma) with test compound (0.1 - 100 µM) dissolved in DMSO (final concentration; less than 0.01%) at 37 °C for 15 min. The reaction was stopped by addition of EDTA (final 7.7 mM) and TxB2 in the supernatant quantitated by using a radioimmunoassay kit (Amersham) according to the manufacturer's procedure.

### Human cell based COX-2 assay

The human sell based COX-2 assay is carried as previously reported by Moore *et al., Inflam. Res.,* Vol. 45, pp. 54- , 1996. Confluent human umbilical vein endothelial cells (HUVECs, Morinaga) in a 96-well U bottom plate are washed with 100µl of RPMI1640 containing 2% FCS and incubation with hIL-1β (final concentration 300U/ml, R & D Systems) at 37°C for 24 hours. After washing, the activated HUVECs are stimulated with A23187 (final concentration 30µM) in Hanks buffer containing 0.2% BSA, 20mM Hepes and a test compound (0.1nM - 100µM) dissolved in DMSSO (final concentration; less than 0.01%) 37°C for 15 minutes. 6-Keto-PGF_{1α}, stable metabolite of PGI₂, in the supernatant is quantitated after adequate dilution by using a radioimmunoassay kit (supplied by Amersham) according to the manufacture's procedures.

### Canine In vitro assays

The following canine cell based COX 1 and COX-2 assays have been reported in Ricketts *et al.,* Evaluation of Selective Inhibition of Canine Cyclooxygenase 1 and 2 by Carprofen and Other Nonsteroidal Anti-inflammatory Drugs, American Journal of Veterinary Research, 59 (11), 1441-1446.

### Protocol for Evaluation of Canine COX-1 Activity

Test drug compounds were solubilized and diluted the day before the assay was to be conducted with 0.1 mL of DMSO / 9.9 mL of Hank's balanced salts solution (HBSS), and stored overnight at 4° C. On the day that the assay was carried out, citrated blood was drawn from a donor dog, centrifuged at 190 x g for 25 min at room temperature, and the resulting platelet-rich plasma was then transferred to a new tube for further procedures. The platelets were washed by centrifuging at 1500 x g for 10 min at room temperature. The platelets were washed with platelet buffer comprising Hank's buffer (Ca free) with 0.2% bovine serum albumin (BSA) and 20 mM HEPES. The platelet samples were then adjusted to 1.5 x 10⁷ / mL, after which 50 µl of calcium ionophore (A23187) together with a calcium chloride solution were added to 50 µl of test drug compound dilution in plates to produce final concentrations of 1.7 µM A23187 and 1.26 mM Ca. Then, 100 µl of canine washed platelets were added and the samples were incubated at 37° C for 15 min, after which the reaction was stopped by adding 20 µl of 77 mM EDTA. The plates were then centrifuged at 2000 x g for 10 min at 4° C, after which 50 µl of supernatant was assayed for thromboxane B₂ (TXB₂) by enzyme-immunoassay (EIA). The pg/mL of TXB₂ was calculated from the standard line included on each plate, from which it was possible to calculate the percent inhibition of COX-1 and the IC₅₀ values for the test drug compounds.

### Protocol for Evaluation of Canine COX-2 Activity

A canine histocytoma (macrophage-like) cell line from the American Type Culture Collection designated as DH82, was used in setting up the protocol for evaluating the COX-2 inhibition activity of various test drug compounds. There was added to flasks of these cells 10 µg/mL of LPS, after which the flask cultures were incubated overnight. The same test drug compound dilutions as described above for the COX-1 protocol were used for the COX-2 assay and were prepared the day before the assay was carried out. The cells were harvested from the culture flasks by scraping, and were then washed with minimal Eagle's media (MEM) combined with 1% fetal bovine serum, centrifuged at 1500 rpm for 2 min, and adjusted to a concentration of 3.2 x 10⁵ cells/mL. To 50 µl of test drug dilution there was added 50 µl of arachidonic acid in MEM to give a 10 µM final concentration, and there was added as well 100 µl of cell suspension to give a final concentration of 1.6 x 10⁵ cells/mL. The test sample suspensions were incubated for 1 hr and then centrifuged at 1000 rpm for 10 min at 4° C, after which 50 µl aliquots of each test drug sample were delivered to EIA plates. The EIA was performed for prostaglandin E₂ (PGE₂), and the pg/mL concentration of PGE₂ was calculated from the standard line included on each plate. From this data it was possible to calculate the percent inhibition of COX-2 and the IC₅₀ values for the test drug compounds. Repeated investigations of COX-1 and COX-2 inhibition were conducted over the course of several months. The results are averaged, and a single COX-1 COX-2 ratio is calculated.

Whole blood assays for COX-1 and COX-2 are known in the art such as the methods described in C. Brideau, et al., A Human Whole Blood Assay for Clinical Evaluation of Biochemical Efficacy of Cyclooxygenase Inhibitors, Inflammation Research, 45, 68-74, (1996). These methods may be applied with feline, canine or human blood as needed.

### In vivo assays

### Carrageenan induced foot edema in rats

Male Sprague-Dawley rats (5 weeks old, Charles River Japan) were fasted overnight. A line was drawn using a marker above the ankle on the right hind paw and the paw volume (V0) was measured by water displacement using a plethysmometer (Muromachi). Animals were given orally either vehicle (0.1% methyl cellulose or 5% Tween 80) or a test compound (2.5 ml per 100 g body weight). One hour later, the animals were then injected intradermally with λ-carrageenan (0.1 ml of 1% w/v suspension in saline, Zushikagaku) into right hind paw (Winter *et al., Proc. Soc. Exp. Biol. Med.*, **111**, 544, 1962; Lombardino *et al., Arzneim. Forsch.*, **25**, 1629, 1975) and three hours later, the paw volume (V3) was measured and the increase in volume (V3-V0) calculated. Since maximum inhibition attainable with classical NSAIDs is 60-70%, ED30 values were calculated.

### Gastric ulceration in rats

The gastric ulcerogenicity of test compound was assessed by a modification of the conventional method (Ezer *et al., J. Pharm. Pharmacol.*, **28,** 655, 1976; Cashin *et al*., *J. Pharm. Pharmacol.*, **29**, 330-336, 1977). Male Sprague-Dawley rats (5 weeks old, Charles River Japan), fasted overnight, were given orally either vehicle (0.1% methyl cellulose or 5% Tween 80) or a test compound (1 ml per 100 g body weight). Six hours after, the animals were sacrificed by cervical dislocation. The stomachs were removed and inflated with 1% formalin solution (10 ml). Stomachs were opened by cutting along the greater curvature. From the number of rats that showed at least one gastric ulcer or haemorrhaging erosion (including ecchymosis), the incidence of ulceration was calculated. Animals did not have access to either food or water during the experiment.

### Data Analysis

Statistical program packages, SYSTAT (SYSTAT, INC.) and StatView (Abacus Cencepts, Inc.) for Macintosh were used. Differences between test compound treated group and control group were tested for using ANOVA. The IC₅₀ (ED₃₀) values were calculated from the equation for the log-linear regression line of concentration (dose) versus percent inhibition.

All compounds prepared in the Working Examples were tested by these methods, and found to have activity to inhibit COX-2.

Also, the above-mentioned most preferred individual compounds were found to show IC₅₀ values of 0.001 µM to 0.5 µM with respect to inhibition of COX-2.

COX-2 selectivity can be determined by ratio in terms of IC₅₀ value of COX-1 inhibition to COX-2 inhibition. In general, it can be said that a compound showing a COX-1/COX-2 inhibition ratio of more than 2 has good COX-2 selectivity.

Some compounds prepared in Examples showed COX-1/COX-2 inhibition ratio of more than 10.

The following examples contain detailed descriptions of the methods of the preparation of compounds of formula (I). These detailed descriptions fall within the scope of the invention and serve to exemplify the above described general synthetic procedures which form part of the invention. These detailed descriptions are presented for illustrative purposes only and are not intended to restrict the scope of the present invention.

### EXAMPLES

The invention is illustrated in the following non-limiting examples in which, unless stated otherwise: all operations were carried out at room or ambient temperature, that is, in the range of 18-25 °C; evaporation of solvent was carried out using a rotary evaporator under reduced pressure with a bath of up to 60 °C; reactions were monitored by thin layer chromatography (tlc) and reaction times are given for illustration only; melting points (m.p.) given are uncorrected (polymorphism may result in different melting points); structure and purity of all isolated compounds were assured by at least one of the following techniques: tlc (Merck silica gel 60 F-254 precoated plates), mass spectrometry, nuclear magnetic resonance (NMR) or microanalysis. Yields are given for illustrative purposes only. Flash column chromatography was carried out using Merck silica gel 60 (230-400 mesh ASTM). Low-resolution mass spectral data (EI) were obtained on a Automass 120 (JEOL) mass spectrometer. Low-resolution mass spectral data (ESI) were obtained on a Quattro II (Micromass) mass spectrometer. NMR data was determined at 270 MHz (JEOL JNM-LA 270 spectrometer) using deuterated chloroform (99.8% D) or dimethylsulfoxide (99.9% D) as solvent unless indicated otherwise, relative to tetramethylsilane (TMS) as internal standard in parts per million (ppm); conventional abbreviations used are: s = singlet, d = doublet, t = triplet, q = quartet, m = multiplet, br = broad, etc.

### EXAMPLE 1

### [6-CHLORO-3-(1H-1,2,3,4-TETRAZOL-5-YLMETHYL)-1H-INDOL-2-YL](4-METHYL-2-PYRIDINYL)METHANONE

### STEP 1.Methyl trans-4-chloro-2-(phenylsulfonylamino)cinnamate

To a solution of methyl *trans*-4-chloro-2-aminocinnamate (R.W.Carling et al., *J.Med.Chem*., **1993**, 36, 3397., 30.7 g, 0.15 mol) and pyridine (36 ml, 0.45 mol) in dichloromethane (500 ml) was added benzenesulfonyl chloride (20 ml, 0.16 mol). After stirring for 20 h, methanol (50 ml) was added and the mixture was concentrated. The residual solids were dissolved in dichloromethane (700 ml) and washed with 2N aqueous HCl (150 ml), brine (150 ml) and dried (MgSO₄). After removal of solvent, the residual solids were recrystallized from ethanol to give 40 g (76 %) of the title compound as pale yellow solids.
¹H-NMR (CDCl₃) δ: 7.77-7.71 (2H, m), 7.59-7.52 (1H, m), 7.48-7.35 (5H, m), 7.20 (1H, dd, J=2.0, 8.4 Hz), 6.85 (1H, br s), 6.15 (1H, d, J=15.8 Hz), 3.78 (3H, s).

### STEP 2. trans-4-Chloro-2-(phenylsulfonylamino)cinnamic acid

To a stirred suspension of methyl *trans*-4-chloro-2-(phenylsulfonylamino)cinnamate (step 1, 20.0 g, 56.8 mmol) in ethanol (300 ml) was added 2N aqueous NaOH (55ml, 110 mmol) at room temperature. The mixture was heated at reflux temperature for 3 h and cooled. The solvent was removed and the residue was dissolved in water (300 ml). After washing with diethyl ether (200 ml), the aqueous layer was acidified with 2N aqueous HCl (60 ml) and extracted with ethyl acetate (300 ml x 2). The combined extracts were washed with brine (200 ml), and dried (MgSO₄). Removal of solvent gave 19.1 g (99%) of the title compound as white solids.
¹H-NMR (DMSO-d₆) δ: 12.41 (1H, s), 10.26 (1H, s), 7.80 (1H, d, J=8.7 Hz), 7.72-7.50 (5H, m), 7.33 (1H, dd, J=2.0, 8.4 Hz), 6.91 (1H, d, J=2.1 Hz), 6.34 (1H, d, J=16.0 Hz).

### STEP 3. (E)-3-{4-Chloro-2-[(phenylsulfonyl)amino]phenyl}-N-(2-cyanoethyl)-2-propenamide

To a stirred solution of *trans*-4-chloro-2-(phenylsulfonylamino)cinnamic acid (step 2, 15.0g, 44 mmol) and 3-aminopropionitrile (3.7g, 53 mmol) in DMF (50 ml) was added dropwise diethyl cyanophosphonate (7.4 ml, 49 mmol) and triethylamine (6.88 ml, 49 mmol) at room temperature. After stirring for 4h, the mixture was poured into water (300 ml) and extracted with ethyl acetate-toluene (2:1, 600 ml x 2). The combined extracts were washed with 10% aquous citric acid (300 ml), water (300 ml), saturated aqueous sodium bicarbonate (300 ml), brine (300 ml), and dried (MgSO₄). Removal of solvent gave 16.8g (81 %) of the title compound as white solids.
¹H-NMR (CDCl₃) δ: 7.97-7.29 (8H, m), 7.13 (1H, dd, J=2.1, 8.4 Hz), 6.16 (1H, d, J=15.8 Hz), 3.56 (2H, dt, J=6.1, 6.3 Hz), 2.69 (2H, t, J=6.3 Hz).

### STEP 4. N-(5-Chloro-2-{(E)-2-[1-(2-cyanoethyl)-1H-1,2,3,4-tetrazol-5 yl]ethenyl}phenyl)benzenesulfonamide

To a stirred solution of (E)-3-{4-chloro-2-[(phenylsulfonyl)amino]phenyl}-N-(2-cyanoethyl)-2-propenamide in (16.7g, 43.0 mmol) in THF (300 ml) was added azidotrimethylsilane (20.7 ml, 156 mmol), diethyl azodicarboxylate (24.5 ml, 156 mmol) and triphenylphosphine (40.9g, 156 mol) at room temperature. After stirring for 2h, the mixture was poured into 10% aqueous solution of ammonium cerium(IV) nitrate (300 ml) and extracted with ethyl acetate (500 ml). The extract was washed with water (300 ml), brine (300 ml), dried (MgSO4), and concentrated. The residual oil was dissolved in dichloromethane (ca. 100 ml), and then n-hexane (ca. 10 ml) was added. The mixture was allowed to stand overnight and the precipitates were collected by filtration to afford 6.23 g (35 %) of the title compound as a white powder.
¹H-NMR (DMSO-d₆) δ: 10.38 (1H, s), 7.99 (1H, d, J=8.6 Hz), 7.94 (1H, d, J=16.0 Hz), 7.72-7.42 (6H, m), 7.31 (1H, d, J=16.0 Hz), 6.99 (1H, d, J=2.3 Hz), 4.85 (2H, t, J=6.6 Hz), 3.21 (2H, t, J=6.6 Hz).

### STEP 5. [6-Chloro-3-(1H-1,2,3,4-tetrazol-5-ylmethyl)-1H-indol-2-yl](4-methyl-2-pyridinyl)methanone

To a stirred solution of N-(5-chloro-2-{(E)-2-[1-(2-cyanoethyl)-1H-1,2,3,4-tetrazol-5-yl]ethenyl}phenyl)benzenesulfonamide (1.0 g, 2.4 mmol) in acetone (50 ml) was added 2-bromoacetyl-4-methylpyridine hydrobromide* (1.42 g, 4.8 mmol) and potassium carbonate (3.3 g, 24 mmol) at room temperature. After stirring for 7h, 1,8-diazabicyclo[5.4.0]undec-7-ene (0.72 ml, 4.8 mmol) was added and the mixture was stirred for an additional 1h. The mixture was filtered and the filtrate was concentrated. The residue was dissolved in THF-methanol (1:1, 30 ml) and 2N aqueous NaOH (10 ml) was added with strring. After 1.5h, the mixture was concentrated and the residue was partitioned between ethyl acetate (200 ml) and 10 % aqueous citric acid (200 ml). The separated aqueous layer was extracted with ethyl acetate (200 ml). The combined organic phases were washed with brine (200 ml), dried (MgSO₄) and concentrated. The residual oil was purified by flash column chromatography eluting with ethyl acetate/n-hexane (2:1) gave 429 mg (51 %) of the title compound as yellow solids.
MS (negative ESI) m/z: 351 [M-H]⁻.
IR (KBr) ν: 1638, 1597, 1524, 1302, 1277, 1200.
¹H-NMR (DMSO-d₆) δ: 12.47 (1H, s), 8.69 (1H, d, J=5.1 Hz), 7.92 (1H, br s), 7.79 (1H, d, J=1.9 Hz), 7.73 (1H, d, J=8.9 Hz), 7.61-7.54 (1H, m), 7.13 (1H, dd, J=1.9, 8.6 Hz), 4.78 (2H, s), 2.45 (3H, s).
* 2-Bromoacetyl-4-methylpyridine hydrobromide was prepared as follows;

To a stirred solution of 2-acetyl-4-methylpyridine (F.H.Case et al, *J. Am. Chem. Soc.,* **1956**, 78, 5842., 7.8 g, 57.7 mmol) in 25% HBr-AcOH (40 ml) was added dropwise a solution of bromine (10.1 g, 63.5 mmol) in AcOH (10 ml) with ice-cooling. After stirring for 1h, diethyl ether (100 m) was added and the precipitates were collected by filtration to give 10.8 g (63 %) of the title compound.
¹H-NMR (DMSO-d₆) δ: 8.71 (1H, d, J=5.1Hz), 8.14 (1H, s), 7.75 (1H, d, J=5.1Hz), 5.07 (2H, s), 2.52 (3H, s).

### EXAMPLES 2 and 3

### {6-Chloro-3-[(2-methyl-2H-1,2,3,4-tetrazol-5-yl)methyl]-1H-indol-2-yl}(4-methyl-2-pyridinyl)methanone and {6-Chloro-3-[(1-methyl-1H-1,2,3,4-tetrazol-5-yl)methyl]-1H-indol-2-yl}(4-methyl-2-pyridinyl)methanone

To a stirred suspension of [6-chloro-3-(1H-1,2,3,4-tetrazol-5-ylmethyl)-1H-indol-2-yl](4-methyl-2-pyridinyl)methanone (Example 1, 361 mg, 1.02 mmol) in acetone (20 ml) was added iodomethane (0.04 ml, 0.6 mmol) and potassium carbonate (1.40g, 10.2 mmol) at room temperature. After stirring for 19h, the mixture was concentrated and the residue was partitioned between water (100 ml) and ethyl acetate (100 ml). The separated organic layer was washed with brine (50 ml), dried (MgSO4) and concentrated. The residual solids were purified on silica eluting with ethyl acetate/n-hexane (3:2) to afford {6-chloro-3-[(2-methyl-2H-1,2,3,4-tetrazol-5-yl)methyl]-1H-indol-2-yl}(4-methyl-2-pyridinyl)methanone (64 mg) and {6-chloro-3-[(1-methyl-1H-1,2,3,4-tetrazol-5-yl)methyl]-1H-indol-2-yl}(4-methyl-2-pyridinyl)methanone (44 mg).
{6-Chloro-3-[(2-methyl-2H-1,2,3,4-tetrazol-5-yl)methyl]-1H-indol-2-yl}(4-methyl-2-pyridinyl)methanone:
m.p.: 211.6 °C.
IR(KBr)ν: 3280, 1643, 1597, 1528, 1302, 1281, 1200 cm⁻¹.
¹H-NMR (CDCl₃) δ: 12.56 (1H, br s), 8.60 (1H, d, J=4.9 Hz), 8.18 (1H, br s), 7.66 (1H, d, J=8.7 Hz), 7.50 (1H, d, J=1.8 Hz), 7.38-7.32 (1H, m), 7.09 (1H, dd, J=1.8, 8.7 Hz), 4.96 (2H, s), 4.24 (3H, s), 2.47 (3H, s).
{6-Chloro-3-[(1-methyl-1H-1,2,3,4-tetrazol-5-yl)methyl]-1H-indol-2-yl}(4-methyl-2-pyridinyl)methanone:
m.p.: 227.6 °C.
IR (KBr) ν: 3283, 1641, 1595, 1522, 1277, 1281, 1190 cm⁻¹.
¹H-NMR (CDCl₃) δ: 12.65 (1H, br s), 8.62 (1H, d, J=4.9 Hz), 8.20-8.10 (1H, m), 7.77-7.69 (1H, m), 7.52-7.48 (1H, m), 7.43-7.36 (1H, m), 7.09 (1H, dd, J=1.8, 8.7 Hz), 5.00 (2H, s), 4.05 (3H, s), 2.50 (3H, s).

### EXAMPLE 4

### [6-CHLORO-3-(1H-1,2,3,4-TETRAZOL-5-YLMETHYL)-1H-INDOL-2-YL](3-METHYL-2-PYRIDINYL)METHANONE

The title compound was prepared according to the procedure described in step 5 of Example 1 from 2-bromoacetyl-3-methylpyridine hydrobromide.*
m.p.: 211-215°C.
MS (EI) m/z: 352 (M⁺).
IR (KBr) ν: 1637, 1524, 1524, 1301, 1196, 1150, 1109.
¹H-NMR (DMSO-d₆) δ: 11.73 (1H, br s), 8.30 (1H, br d, J=4.3 Hz), 7.60 (1H, br d, J=7.7Hz), 7.52 (1H, d, J=8.7Hz), 7.40 (1H, d, J=2.0Hz), 7.34 (1H, dd, J=4.61, 7.75Hz), 6.95 (1H, dd, J=1.98, 8.73Hz), 4.25 (2H, s), 2.07 (3H, s).
*2-Bromoacetyl-3-methylpyridine hydrobromide was prepared from 2-acetyl-3-methylpyridine (T.A.Crabb et al., *Org. Magn. Reson.,* **1982,** 20, 242) according to the procedure for preparing 2-bromoacetyl-4-methylpyridine hydrobromide described in step 5 of Example 1.
¹H-NMR (DMSO-d₆) δ: 8.56 (1H, d, J=3.6Hz), 7.84 (1H, d, J=7.7Hz), 7.56-7.60 (1H, m), 5.01 (2H, s), 4.01 (3H, s).

### EXAMPLE 5

### [6-CHLORO-3-(1H-1,2,3,4-TETRAZOL-5-YLMETHYL)-1H-INDOL-2-YL](5-METHYL-2-PYRIDINYL)METHANONE

The title compound was prepared according to the procedure described in step 5 of Example I from 2-bromoacetyl-5-methylpyridine hydrobromide.*
m.p.: 252-254°C.
MS (EI) m/z: 352 (M⁺).
IR (KBr) v: 1647, 1529, 1307, 1242, 1201, 1143.
¹H-NMR (DMSO-d₆) δ: 12.30 (1H, br s), 8.55 (1H, br s), 7.90 (1H, d, J=7.9Hz), 7.80 (1H, br d, J=8.2Hz), 7.67 (1H, d, J=1.7Hz), 7.60 (1H, d, J=8.7Hz), 7.01 (1H, dd, J=1.7, 8.6Hz), 4.66 (2H, s), 2.35 (3H, S).
* 2-Bromoacetyl-5-methylpyridine was prepared as follows;

A mixture of 2-bromo-5-methylpyridine (5.00 g, 29.06 mmol), tri-n-butyl(1-ethoxyvinyl)tin (10.49 g, 29.07 mmol), and tetrakis(triphenylphosphine)palladium (3.36 g, 2.91 mmol) in toluene (40 ml) was heated at reflux temperature for 18 h. The mixture was cooled, filtered through a pad of Celite and then concentrated. The residue (∼10 g) was dissolved in a mixture of THF (100 ml) and water (20 ml), cooled to 0 °C and *N*-bromosuccinimide (5.43 g, 30.52 mmol) was added over 20 min. The resulting mixture was stirred for 0.5 h at the same temperature and then concentrated to ca. 20 ml. The mixture was diluted in ethyl acetate (300 ml), washed with water (100 ml x 3), and dried (MgSO₄). After removal of solvent, the crude product was purified by flash column chromatography eluting with ethyl acetate/hexane (1:15 to 1:10) to afford 2.30 g (37%) of the title compound as an oil.
¹H-NMR (CDCl₃) δ: 8.49 (1 H, br s), 8.01 (1 H, d, J=8.1 Hz), 7.68-7.64 (1 H, m), 4.84 (2 H, s), 2.44 (3 H, s).

### EXAMPLE 6

### [6-CHLORO-3-(1H-1,2,3,4-TETRAZOL-5-YLMETHYL)-1H-INDOL-2-YL](6-METHYL-2-PYRIDINYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (step 4 of Example 1) and 2-bromoacetyl-6-methylpyridine hydrobromide (H.Erlenmeyer, J.Jenni, and B.Prijs, *J.Med.Pharm.Chem*., **1961**, 3, 561-566).
MS (EI) m/z : 352 (M⁺).
IR(KBr)ν: 3234, 2849, 1647, 1528, 1311, 1229, 1205, 1146, 669.
¹H-NMR (DMSO-d₆) δ: 12.21 (1H, s), 7.97 (1H, t, J=7.7 Hz), 7.84 (1H, d, J=7.7 Hz), 7.79 (1H, d, J=2.0 Hz), 7.71 (1H, d, J=8.7 Hz), 7.59 (1H, d, J=7.7 Hz), 7.14 (1H, dd, J=2.0, 8.7 Hz), 4.74 (2H, s), 2.65 (3H, s).

### EXAMPLE 7

### [6-CHLORO-3-(1H-1,2,3,4-TETRAZOL-5-YLMETHYL)-1H-INDOL-2-YL](4-ETHYL-2-PYRIDINYL )METHANONE

The title compound was prepared according to the procedure described in step 5 of Example 1 from N-(5-chloro-2-{(E)-2-[1-(2-cyanoethyl)-lH-1,2,3,4-tetrazol-5-yl]ethenyl}phenyl)benzenesulfonamide (Example 1, step 4) and 2-bromoacetyl-4-ethylpyridine.*
IR (KBr) ν: 3267, 1647, 1595, 1528, 1196, 1059 cm⁻¹.
¹H-NMR (DMSO-d₆) δ: 12.46 (1H, s), 8.71 (1H, d, J=4.9Hz), 7.94 (1H, s), 7.80 (1H, br), 7.73 (1H, d, J=8.9 Hz), 7.61 (1H, br d, J=5.4 Hz), 7.13 (1H, dd, J=1.9, 8.6 Hz), 4.78 (2H, s), 2.76 (2H, q, J=7.3 Hz), 1.23 (3H, t, J=7.6 Hz).
* 2-Bromoacetyl-4-ethylpyridine was prepared as follows;

To a solution of 2-acetyl-4-ethylpyridine (E. C. Constable et al., *J. Am. Chem. Soc.,* **1997**, 119, 5606., 8.37 g, 56.1 mmol) in 25% hydrobromic acid-acetic acid (150 ml) was added dropwise a solution of bromine (9.86 g, 61.7 mmol) in acetic acid (30 ml) with ice-cooling. The mixture was allowed to warm to room temperature and stirred for 2h. Diethyl ether (500 ml) was added to the mixture and the resulting mixture was cooled with an ice-bath. A brown oil, separated out from the solution, was collected by decantation. The oil was treated with saturated aqueous sodium bicarbonate (50 ml) and extracted with diethyl ether (300 ml). The organic layer was dried (MgSO₄) and concentrated to give 15.3 g (88%) of the title compound.
¹H-NMR (CDCl₃) δ: 8.56 (1 H, d, J=5.1 Hz), 7.95 (1 H, br s), 7.36-7.34 (1 H, m), 4.86 (2 H, s), 2.74 (2 H, q, J=7.7 Hz), 1.29 (3 H, t, J=7.6 Hz).

### EXAMPLE 8

### [6-CHLORO-3-(1H-1,2,3,4-TETRAZOL-5-YLMETHYL)-1H-INDOL-2-YL](2-PYRIDINYL)METHANONE

The title compound was prepared according to the procedure described in step 5 of Example 1 from N-(5-chloro-2-{(E)-2-[1-(2-cyanoethyl)-lH-1,2,3,4-tetrazol-5-yl]ethenyl}phenyl)benzenesulfonamide (Example 1, step 4) and 2-bromoacetylpyridine hydrobromide (H.McKennis et al., *J.Org.Chem*., **1963,** 28, 383).
MS (negative ESI) m/z: 337 [M-H]⁻.
¹H-NMR (DMSO-d₆) δ: 12.50 (1H, s), 8.85-8.77 (1H, m), 8.31 (1H, d, J=7.8 Hz), 8.00 (1H, ddd, J=2.2, 7.8, 7.8 Hz), 7.70-7.55 (3H, m), 7.11 (1H, dd, J=1.6, 8.6 Hz), 4.94 (2H, s).

### EXAMPLE 9

### [6-CHLORO-3-(1H-1,2,3,4-TETRAZOL-5-YLMETHYL)-1H-INDOL-2-YL](4-CHLORO-2-PYRIDINNL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (step 4 of Example 1) and 2-bromoacetyl-4-chloropyridine hydrobromide*.
MS (EI) m/z: 372 (M⁺).
IR (KBr) ν: 3294, 2872, 1639, 1568, 1555, 1526, 1234, 1060, 739.
¹H-NMR (DMSO-d₆) δ: 12.32 (1H, s), 8.79 (1H, d, J=5.3 Hz), 8.08 (1H, d, J=2.0 Hz), 7.90 (1H, dd, J=2.0, 5.3 Hz), 7.77-7.71 (2H, m), 7.14 (1H, dd, J=1.6, 8.7 Hz), 4.77 (2H, s).
* 2-Bromoacetyl-4-chloropyridine hydrobromide was prepared as follows;
4-Chloro-2-pyridinecarbonitrile: To a mixture of 4-chloropyridine-*N*-oxide (5.00 g, 38.6 mmol) and trimethylsilyl cycanide (4.84 g, 46.3 mmol) in dichloromethane (60 ml) cooled to 0 °C was added dropwise *N*,*N*-dimethylcarbamoyl chloride (3.8 ml, 40.5 mmol). The mixture was allowed to warm to ambient temperature and stirred for 16 h. The mixture was cooled to 0 °C and a 30% aqueous solution of potassium carbonate (100 ml) was added. The crude product was extracted with dichloromethane (100 ml x 2), the organic extracts dried (MgSO₄) and evaporated to give 4-chloro-2-pyridinecarbonitrile (5.35 g, 100%).
¹H-NMR (CDCl₃) 8.63 (1 H, d, J=4.8 Hz), 7.72 (1 H, d, J=2.6 Hz), 7.55 (1 H, dd, J=1.8, 5.1 Hz).
2-Acetyl-4-chloropyridine: To a solution of 4-chloro-2-pyridinecarbonitrile (5.35 g, 38.6 mmol) in benzene (50 ml) and ether (50 ml) cooled to 0 °C was added dropwise over 20 min a 2M solution of MeMgI in ether (23 ml, 46.3 mmol). After 0.5 h, the mixture was allowed to warm to ambient temperature, and stirring continued for 2 hours. The mixture was cooled to 0 °C and 2M aqueous HCl (100 ml) added. The mixture was made basic with saturated aqueous sodium bicarbonate (∼80 ml) and the organic layer separated and dried (MgSO₄). After removal of solvent, the residue was purified by flash chromatography eluting with ethyl acetate/hexane (1:5) to afford 3.60 g (60%) of 2-acetyl-4-chloropyridine.
¹H-NMR (DMSO-d₆) 8.59 (1 H, d, J=5.1 Hz), 8.04 (1 H, d, J=1.8 Hz), 7.47 (1 H, dd, J=1.8, 5.1 Hz), 2.72 (3 H, s).
2-Bromoacetyl-4-chloropyridine hydrobromide: 2-(Bromoacetyl)-4-chloropyridine hydrobromide was prepared from 2-acetyl-4-chloropyridine according to the method of H. McKennis, Jr., L. B. Turnbull, E. R. Bowman, and E. Tamaki (in *J. Org. Chem.,* **1963**, 28, 383-387).
¹H-NMR (DMSO-d₆) 8.74 (1 H, d, J=5.5 Hz), 8.05 (1 H, d, J=1.8 Hz), 7.88 (1 H, dd, J=2.2 and 5.5 Hz), 5.02 (2 H, s).

### EXAMPLE 10

### [6-CHLORO-3-(1H-1,2,3,4-TETRAZOL-5-YLMETHYL)-1H-INDOL-2-YL](5-CHLORO-2-PYRIDINYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (Example 1, step 4) and 2-bromoacetyl-5-chloropyridine hydrobromide*.
MS (EI) m/z : 372 (M⁺).
IR (KBr) ν: 3267, 2689, 1643, 1526, 1306, 1229, 1204, 1146, 1113, 1016.
¹H-NMR (DMSO-d₆) δ: 12.19 (1H, s), 8.82 (1H, d, J=2.3 Hz), 8.23 (1H, dd, J=2.3, 8.4 Hz), 8.06 (1H, d, J=8.4 Hz), 7.77-7.70 (2H, m), 7.17-7.10 (1H, m), 4.76 (2H, s).
* 2-Bromoacetyl-5-chloropyridine was prepared from 2-bromo-5-chloropyridine (Case, *J. Am. Chem. Soc.,* **1946,** 68, 2574) according to the procedure for preparing 2-bromoacetyl-5-methylpyridine described in Example 5.
¹H-NMR (CDCl₃) δ: 8.63 (1 H, dd, J=0.6, 2.5 Hz), 8.06 (1 H, dd, J=0.6, 8.4 Hz), 7.85 (1 H, dd, J=2.3, 8.4 Hz), 4.79 (2 H, s).

### EXAMPLE 11

### [6-CHLORO-3-(1H-1,2,3,4-TETRAZOL-5-YLMETHYL)-1H-INDOL-2-YL](5-TRIFLUOROMETHYL-2-PYRIDINYL)METHANONE

The title compound was prepared according to the procedure described in step 5 of Example 1 from 2-bromoacetyl-5-trifluoromethylpyridine.*
m.p.: 253-256°C.
MS (EI) m/z : 406 (M⁺).
IR (KBr) ν: 1645, 1525, 1330, 1307, 1204, 1170, 1126, 1076.
¹H-NMR (DMSO-d₆) δ: 12.08 (1H, br s), 9.04 (1H, br s), 8.40 (1H, dd, J=1.7, 8.2Hz), 7.64 (1H, d, J=8.7Hz), 7.57 (1H, dd, J=2.0, 8.7Hz), 4.64 (2H, s).
*2-Bromoacetyl-5-(trifluoromethyl)pyridine was prepared from 2-chloro-5-(trifluoromethyl)pyridine according to the procedure for preparing 2-bromoacetyl-5-methylpyridine described in Example 5.
¹H-NMR (CDCl₃) δ: 8.96 (1 H, br s), 8.23 (1 H, br d, J=8.2 Hz), 8.13 (1 H, dd, J=2.1, 8.1 Hz), 4.83 (2 H, s).

### EXAMPLE 12

### [6-CHLORO-3-(1H-1,2,3,4-TETRAZOL-5-YLMETHYL)-1H-INDOL-2-YL](4-METHOXY-2-PYRIDINYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (step 4 of Example 1) and 2-bromoacetyl-4-methoxypyridine hydrobromide*.
MS (EI) m/z: 368 (M⁺).
IR (KBr) ν: 3220, 2874, 1641, 1591, 1523, 1307, 1138, 1007, 795.
¹H-NMR (DMSO-d₆) δ: 12.52 (1H, s), 8.65 (1H, d, J=5.8 Hz), 7.80 (1H, d, J=1.8 Hz), 7.74 (1H, d, J=8.6 Hz), 7.59 (1H, d, J=2.6 Hz), 7.31 (1H, dd, J=2.6, 5.8 Hz), 7.13 (1H, dd, J=1.8, 8.6 Hz), 4.79 (2H, s), 3.94 (3H, s).
*2-Bromoacetyl-4-methoxypyridine hydrobromide was prepared from 2-acetyl-4-methoxypyridine (B.Case et al., *J. Org. Chem.,* **1961,** 26, 4415) according to the procedure for preparing 2-bromoacetyl-4-methylpyridine hydrobromide described in step 5 of Example 1.
¹H-NMR (DMSO-d₆) δ: 8.59-8.62 (1H, m), 7.63-7.65 (1H, m), 7.33-7.37 (1H, m), 5.03 (2H, s), 3.96 (3H, s).

### EXAMPLE 13

### [6 -CHLORO-3-(1H-1,2,3,4-TETRAZOL-5-YLMETHYL)-1H-INDOL-2-YL](4-PYRDINYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (Example 1, step 4) and 4-bromoacetylpyridine hydrobromide (L. W. Deady, M. S. Stanborough, *Aust. J. Chem.*, **1981**, 34, 1295).
MS (EI) m/z: 338 (M⁺).
IR (KBr) ν: 3246, 2870, 1637, 1526, 1439, 1323, 1248, 943, 841.
¹H-NMR (DMSO-d₆) δ: 11.92 (1H, s), 8.80 (2H, d, J=4.5 Hz), 7.68 (1H, d, J=8.7 Hz), 7.63 (2H, d, J=4.5 Hz), 7.51 (1H, d, J=1.8 Hz), 7.15 (1H, dd, J=1.8, 8.7 Hz), 4.58 (2H, s).

### EXAMPLE 14

### [6-CHLORO -3-(1H-1,2,3,4-TETRAZOL-5-YLMETHYL)-1H-INDOL-2-YL](4-CHLOROPHENYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (Example 1, step 4) and 2-bromo-4'-chloroacetophenone.
MS (EI) m/z: 371(M⁺).
IR (KBr) ν: 1643, 1589, 1541, 1294, 1256, 1092 cm⁻¹.
¹H-NMR (CDCl₃) δ: 8.85 (1H, br s), 7.84 (2H, d, J=8.4 Hz), 7.80 (1H, d, J=8.9 Hz), 7.54 (2H, d, J=8.6 Hz), 7.37 (1H, d, J=1.6 Hz), 7.19 (1H, dd, 1.9, 8.6 Hz), 4.63 (2H, s).

### EXAMPLE 15

### [6-CHLORO-3-(1H-1,2,3,4-TETRAZOL-5-YLMETHYL)-1H-INDOL-2-YL](3-METHOXYMETHYL-2-FURYL) METHANONE

The title compound was prepared according to the procedure described in step 5 of Example 1 from 2-bromoacetyl-3-(methoxymethyl)furan.*
m.p.: 227-229°C.
MS (EI) m/z: 371 (M⁺).
IR (KBr) ν: 1578, 1523, 1475, 1406, 1340, 1259.
¹H-NMR (DMSO-d₆) δ: 11.86 (1H, br s), 8.05 (1H, d, J=1.7Hz), 7.68 (1H, d, J=8.7Hz), 7.63 (1H, d, J=1.5Hz), 7.13 (1H, dd, J=1.98, 8.73Hz), 6.86 (1H, d, J=1.48Hz), 4.72 (2H, s), 4.67 (2H, s), 3.34 (3H, s).
*2-Chloroacetyl-3-(methoxymethyl)furan

To a solution of 3-(methoxymethyl)furan (N.Greeves et al., *Synthesis*, **1993**, 1109, 2.33 g, 20.78 mmol) in THF (50 ml) was added 1.55M n-butyl lithium in hexane (16.1 ml, 24.94 mmol) at -78°C and the mixture was stirred at that temperature for 2h. The resulting mixture was warmed slowly to 0°C and 2-chloro-*N*-methoxy-*N*-methylacetamide (5.72 g, 41.56 mmol) was added. The mixture was quenched with saturated aqueous ammonium chloride (50 ml) and the organic layer was separated. The organic layer was washed with water (50 ml x 2), brine (50 ml), dried (MgSO₄), and concentrated. The residue was purified by flash chromatography eluting with ethyl acetate/hexane (1:4) to afford 1.43 g (37%) of the title compound as an oil.
¹H-NMR (CDCl₃) δ: 7.52 (1H, d, J=1.8Hz), 6.74 (1H, d, J=1.8Hz), 4.73 (2H, s), 4.62 (2H, s), 3.44 (3H, s).

### EXAMPLE 16

### [6-CHLORO-3-(1H-1,2,3,4-TETRAZOL-5-YLMETHYL)-1H-INDOL-2-YL](2-THIENYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (step 4 of Example 1) and 2-bromoacetylthiazole hydrobromide (A.Dondoni, A.Marra, and P.Merino, *J.Am.Chem.Soc.*, **1994**, 116, 3324).
MS (EI) m/z : 344 (M⁺).
IR (KBr) ν: 3285, 2696, 1636, 1533, 1327, 1140, 1053, 793.
¹H-NMR (DMSO-d₆) δ: 12.19 (1H, s), 8.37-8.31 (2H, m), 7.88 (1H, d, J=2.0 Hz), 7.76 (1H, d, J=8.7 Hz), 7.15 (1H, dd, J=2.0, 8.7 Hz), 4.85 (2H, s).

### EXAMPLE 17

### [6-CHLORO-3-(1H-1,2,3,4-TETRAZOL-5-YLMETHYL)-1H-INDOL-2-YL](5-METHYL-2-THIENYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (Example 1, step 4) and 2-bromoacetyl-5-methylthiazole hydrobromide*.
MS (EI) m/z: 358(M⁺).
IR (KBr) ν: 3279, 2853, 1636, 1533, 1400, 1315, 1269, 1138, 800.
¹H-NMR (DMSO-d₆) δ: 12.12 (1H, s), 8.05 (1H, s), 7.90-7.85 (1H, m), 7.74 (1H, d, J=8.9 Hz), 7.19-7.11 (1H, m), 4.84 (2H, s), 2.63 (3H, s).
* 2-Bromoacetyl-5-methylthiazole was prepared from 2-acetyl-5-methylthiazole (Metzger et al., *Bull. Soc. Chim. Fr.*, **1953,** 702) according to the method of H.McKennis, Jr., L.B.Turnbull, E.R.Bowman, and E.Tamaki (in *J.Org.Chem*., **1963,** 28, 383-387).
¹H-NMR (DMSO-d₆) δ: 7.91 (1 H, d, J=1.2 Hz), 4.87 (2 H, s), 2.58 (3 H, d, J=0.8 Hz).

### EXAMPLES 18 and 19

### {6 -CHLORO-3-[(1-METHYL-1H-1,2,3,4-TETRAZOL-5 -YL)METHYL]-1H-INDOL-2-YL}(5-METHYL-2-THIENYL)METHANONE AND {6-CHLORO-3-[(2-METHYL-2H-1,2,3,4-TETRAZOL-5-YL)METHYL]-1H-INDOL-2-YL}(5-METHYL-2-THIENYL)METHANONE

The title compounds were prepared according to the procedure described in Example 2 and Example 3 from [6-chloro-3-(1H-1,2,3,4-tetrazol-5-ylmethyl)-1H-indol-2-yl](5-methyl-2-thienyl)methanone (Example 17).
{6-chloro-3-[(1-methyl-1H-1,2,3,4-tetrazol-5-yl)methyl]-1H-indol-2-yl}(5-methyl-2-thienyl)methanone:
MS (El) m/z: 372 (M⁺).
¹H-NMR (CDCl₃) δ: 11.82 (1H, s), 7.82-7.73 (2H, m), 7.52-7.48 (1H, m), 7.12 (1H, dd, J=1.8, 8.9 Hz), 4.99 (2H, s), 4.05 (3H, s), 2.64 (3H, s).
{6-chloro-3-[(2-methyl-2H-1,2,3,4-tetrazol-5-yl)methyl]-1H-indol-2-yl}(5-methyl-2-thienyl)methanone:
MS (EI) m/z : 372 (M⁺).
¹H-NMR (CDCl₃) δ: 11.74 (1H, s), 7.74 (1H, s), 7.65 (1H, d, J=8.7 Hz), 7.48 (1H,d, J=1.6 Hz), 7.09 (1H, dd, J=1.6, 8.7 Hz), 4.94 (2H, s), 4.25 (3H, s), 2.61 (3H, s).

### EXAMPLE 20

### [6-CHLORO-3-(1H-1,2,3,4-TETRAZOL-5-YLMETHYL)-1H-INDOL-2-YL](1-METHYL-2-IMIDAZOLYL)METHANONE

The title compound was prepared according to the procedure described in step 5 of Example I from N-(5-chloro-2-{(E)-2-[1-(2-cyanoethyl)-1H-1,2,3,4-tetrazol-5-yl]ethenyl}phenyl)benzenesulfonamide (Example 1, step 4) and 2-bromoacetyl-1-methyl-1H-imidazol hydrobromide*.
MS (EI) m/z: 341(M⁺).
IR (KBr) ν: 3250, 1624, 1528, 1402, 1146, 1053, 999, 792.
¹H-NMR (DMSO-d₆) δ: 12.35 (1H, s), 7.88 (1H, d, J=2.0 Hz), 7.69 (1H, d, J=8.7 Hz), 7.66 (1H, s), 7.35 (1H, s), 7.11 (1H, dd, J=2.0, 8.7 Hz), 4.84 (2H, s), 4.00 (3H, s).
*2-bromoacetyl-1-methy-1H-limidazole hydrobromide was prepared from 2-acetyl-1-methyl-1H-imidazole according to the procedure for preparing 2-bromoacetyl-4-methyl-1H-pyridine hydrobromide described in step 5 of Example 1.
¹H-NMR (DMSO-d₆) δ: 7.69 (1H, s), 7.27 (1H, s), 4.68 (2H, s), 3.81 (3H, s).

### EXAMPLE 21

### [6-CHLORO-3-(1H-1,2,3,4-TETRAZOL-5-YLMETHYL)-1H-INDOL-2-YL](3-ISOQUINOLINYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (Example 1, step 4) and 3-bromoacetylisoquinoline hydrobromide*.
MS (EI) m/z 388 (M⁺).
IR (KBr) ν: 3124, 2866, 1639, 1616, 1521, 1200, 791.
¹H-NMR (DMSO-d₆) δ: 12.59 (1H, s), 9.54 (1H, s), 8.67 (1H, s), 8.42-8.34 (1H, m), 8.30-8.22 (1H, m), 7.98-7.87 (2H, m), 7.82 (1H, d, J=2.0 Hz), 7.75 (1H, d, J=8.7 Hz), 7.15 (1H, dd, J=2.0, 8.7 Hz), 4.83 (2H, s).
* 3-Bromoacetylisoquinoline hydrobromide was prepared from 3-acetylisoquinoline (D. L. Klayman et al., *Arzneim. Forsch*., **1986**, 36, 10) according to the method of H.McKennis, Jr., L.B.Turnbull, E.R.Bowman, and E.Tamaki (in *J.Org.Chem*., **1963**, 28, 383-387).
¹H-NMR (DMSO-d₆) δ: 9.49(1 H, s), 8.68 (1 H, s), 8.34-8.26 (2 H, m), 7.99-7.88 (2 H, m), 5.14 (2 H, s).

### EXAMPLE 22

### [6-CHLORO-3-(1H-1,2,3,4-TETRAZOL-5-YLMETHYL)-1H-INDOL-2-YL](2-QUINOLINYL)METHANONE

The title compound was prepared according to the procedure described in step 5 of Example 1 from N-(5-chloro-2-{(E)-2-[1-(2-cyanoethyl)-1H-1,2,3,4-tetrazol-5-yl]ethenyl}phenyl)benzenesulfonamide (step 4 of Example 1) and 2-bromoacetylquinoline hydrobromide*.
MS (EI) m/z: 388(M⁺).
IR (KBr) ν: 3267, 1641, 1528, 1315, 1148, 773.
¹H-NMR (DMSO-d₆) δ: 12.44 (1H, s), 8.65 (1H, d, J=8.6 Hz), 8.45 (1H, d, J=8.4 Hz), 8.15 (1H, d, J=8.4 Hz), 8.13 (1H, d, J=8.1 Hz), 8.00-7.92 (1H, m), 7.87 (1H, d, J=1.8 Hz), 7.86-7.78 (1H, m), 7.76 (1H, d, J=8.7 Hz), 7.16 (1H, dd, J=1.8, 8.7 Hz), 4.81 (2H, s).
* 2- bromoacetylquinoline hydrobromide was prepared as follows;
2-Acetylquinoline: To a stirred solution of 2-quinolinecarbonitrile (5.0 g, 32.4 mmol) in benzene (60 ml)-diethyl ether (40 ml) was added dropwise MeMgI (2M in ether, 21 ml) at 0°C under nitrogen atmosphere, and the mixture was stirred at room temperature for 2.5 hours. The mixture was hydrolyzed with 2N HCl (40 ml) with stirring for 0.5h and the organic layer was separated. The aqueous layer was made basic with 2N NaOH, and extracted with diethyl ether (100 ml). The combined organic layers were washed with brine, dried over MgSO₄, and concentrated to give 5.17 g (93%) of the title compound as yellow solids.
¹H-NMR (CDCl₃) d: 8.27 (1H, d, J=8.4 Hz), 8.24-8.18 (1H, m), 8.14 (1H, d, J=8.4 Hz), 7.91-7.85 (1H, m), 7.83-7.75 (1H, m), 7.69-7.61 (1H, m), 2.88 (3H, s).
2- Bromoacetylquinoline hydrobromide: The title compound was prepared according to the procedure for preparing 2-bromoacetyl-4-methylpyridine hydrobromide described in step 5 of Example 1 from 2-acetylquinoline.
¹H-NMR (DMSO-d₆) d: 8.63 (1H, d, J=8.7 Hz), 8.22-8.08 (3H, m), 7.97-7.88 (1H, m), 7.84-7.76 (1H, m), 5.23 (2H, s).

### EXAMPLE 23

### STEP 1. Methyl trans-5-chloro-2-nitrocinnamate

A mixture of the 5-chloro-2-nitrobenzaldehyde (9.68 g, 52.16 mmol) and triphenylphophoranylidene acetate (18.31 g, 54.77 mmol) in toluene (200 ml) was heated at reflux temperature for 2 hours. The mixture was concentrated and the crystalline residue was purified by flash column chromatography eluting with ethyl acetate/hexane (1:5) to afford crystals. Recrystallization from ethyl acetate/hexane gave 7.54 g (60%) of the title compound as pale yellow solids.
¹H-NMR (CDCl₃) δ: 8.09 (1 H, d, J=15.8 Hz), 8.04 (1 H, d, J=8.7 Hz), 7.60 (1 H, d, J=2.1 Hz), 7.51 (1 H, dd, J=2.1, 8.7 Hz), 6.36 (1 H, d, J=15.8 Hz), 3.84 (3 H, s).

### STEP 2. Methyl trans-2-amino-5-chlorocinnamate

A mixture of methyl 5-chloro-2-nitrocinnamate (step 1, 3.00 g, 12.42 mmol), iron powder (3.65 g, 62.08 mmol), ammonium chloride (332 mg, 6.21 mmol), ethanol (60 ml) and water (10 ml) was heated at reflux temperature for 2 hours. The mixture was cooled and filtered through a pad of Celite. The filtrate was concentrated. The residue was diluted with ethyl acetate (200 ml) and washed with water (100 ml x 2). After drying (MgSO₄), removal of solvent gave 2.57 g (98%) of the title compound as crystals.
¹H-NMR (CDCl₃) δ: 7.73 (1 H, d. J=15.8 Hz), 7.34 (1 H, d, J=2.5 Hz), 7.12 (1 H, dd, J=2.3, 8.6 Hz), 6.64 (1 H, d, J=8.6 Hz), 6.35 (1 H, d, J=15.8 Hz), 3.95 (2 H, br s), 3.81 (3 H, s).

### STEP 3. Methyl trans-5-chloro-2-(phenylsulfonylamino)cinnamate

The title compound was prepared according to the procedure described in step of step 1 of Example 1 from methyl *trans*-2-amino-5-chlorocinnamate (step 2).
¹H-NMR (CDCl₃) δ: 7.72-7.67 (2 H, m), 7.58-7.51 (1 H, m), 7.47-7.40 (4 H, m), 7.36 (1 H, d, J=8.6 Hz), 7.31 (1 H, dd, J=2.1, 8.6 Hz), 6.14 (1 H, d, J=15.8 Hz), 3.78 (3 H, s). One signal due to NH was not observed.

### STEP 4. trans-5-Chloro-2-(phenylsulfonylamino)cinnamic acid

The title compound was prepared according to the procedure described in step of step 2 of Example 1 from methyl *trans*-5-chloro-2-(phenylsulfonylamino)cinnamate (step 3).
¹H-NMR (DMSO-d₆) δ: 10.12 (1H, s), 7.85 (1H, d, J=2.4 Hz), 7.75-7.48 (6H, m), 7.37 (1H, dd, J=2.2, 8.4 Hz), 6.91 (1H, d, J=8.4 Hz), 6.42 (1H, d, J=16.2 Hz).

### STEP 5. (E)-3-{5-Chloro-2-[(phenylsulfonyl)amino]phenyl}-N-(2-cyanoethyl)-2-propenamide

The title compound was prepared according to the procedure described in step of step 3 of Example 1 from *trans*-5-chloro-2-(phenylsulfonylamino)cinnamic acid (step 4).
¹H-NMR (CDCl₃) δ: 9.25 (1H, br s), 7.89 (1H, dd, J=6.1, 6.1 Hz), 7.66-7.59 (2H, m), 7.44-7.26 (5H, m), 7.15 (1H, dd, J=2.3, 8.6 Hz), 6.12 (1H, d, J=16.0 Hz), 3.54-3.44 (2H, m), 2.62 (2H, t, J=6.2).

### STEP 6. N-(5-Chloro-2-{(E)-2-[1-(2-cyanoethyl)-1H-1,2,3,4-tetrazol -5-yl]ethenyl}phenyl)benzenesulfonamide

The title compound was prepared according to the procedure described in step 4 of Example 1 from (E)-3-{5-chloro-2-[(phenylsulfonyl)amino]phenyl}-N-(2-cyanoethyl)-2-propenamide (step 5).
¹H-NMR (DMSO-d₆) δ: 10.24 (1H, s), 8.07 (1H, d, J=2.43), 7.93 (1H, d, J=16.2 Hz), 7.70-7.30 (7H, m), 6.97 (1H, d, J=8.6 Hz), 4.87 (2H, t, J=6.5 Hz), 3.22 (2H, t, J=6.2 Hz).

### STEP 7. [5-Chloro-3-(1H-1,2,3,4-tetrazol-5-ylmethyl)-1H-indol-2-yl](4-methyl-2-pyridinyl)methanone

The title compound was prepared according to the procedure described in step of step 5 of Example 1 from N-(5-chloro-2-{(E)-2-[1-(2-cyanoethyl)-1H-1,2,3,4-tetrazol-5-yl]ethenyl}phenyl)benzenesulfonamide (step 6).
MS (negative ESI) m/z: 351 [M-H]⁻.
IR (KBr) ν: 3254, 1647, 1595, 1526, 1275, 1202, 1059 cm⁻¹.
¹H-NMR (DMSO-d₆) δ: 12.60 (1H, s), 8.64 (1H, d, J=4.9 Hz), 8.17-8.12 (1H, m), 7.73-7.68 (1H, m), 7.48 (1H, d, J=8.9 Hz), 7.43-7.38 (1H, m), 7.31 (1H, dd, J=1.9, 8.9 Hz), 4.90 (2H, s), 2.50 (3H, s).

### EXAMPLE 24

### [6-TRIFLUOROMETHYL-3-(1H-1,2,3,4-TETRAZOL-5-YLMETHYL)-1H-INDOL-2 -YL1 (4-METHYL-2-PYRIDINYL )METHANONE

### STEP 1. Methyl trans-2-amino-4-(trifluoromethyl)cinnamate

A mixture of 2-bromo-5-(trifluoromethyl)aniline (2.0 g, 8.33 mmol), methyl acrylate (1.9 ml, 20.8 mmol), palladium(II) acetate (224 mg, 1.00 mmol), tri-o-tolylphosphine (1.2 g, 4.0 mmol), and triethylamine (4.5 ml) in acetonitrile (17 ml) was heated at 110 °C. After stirring for 2 h, methyl acrylate (1.0 ml, 10.4 mmol), palladium(II) acetate (112 mg, 0.50 mmol), tri-*o*-tolylphosphine (0.60 g, 2.0 mmol), triethylamine (2.3 ml) were added and the mixture was stirred at 110 °C for 7 hours. The solvent was removed and the residue was diluted with ethyl acetate (100 ml), washed with water (100 ml), dried (MgSO₄) and concentrated. The residue was purified by flash column chromatography eluting with ethyl acetate/hexane (1:5/1:4) to afford 1.16 g (57 %) of the title compound as yellow solids.
¹H-NMR (CDCl₃) δ: 7.79 (1H, d, J=15.8Hz), 7.46-7.43 (1H, m), 7.00-6.94 (2H, m), 6.41 (1H, d, J=15.8Hz), 4.18 (2H, m), 3.82 (3H, s).

### STEP 2. Methyl trans-2-phenylsulfonylamino-4-(trifluoromethyl )cinnamate

The title compound was prepared according to the procedure described in step 1 of Example 1 from methyl *trans*-2-amino-4-(trifluoromethyl)cinnamate (step 1).
¹H-NMR (CDCl₃) δ: 7.77-7.68 (2H, m), 7.65-7.53 (4H, m), 7.47-7.41 (3H, m), 6.24 (1H, d, J=15.8Hz), 3.80 (3H, s).

### STEP 3. trans -4-Trifluoromethyl-2 - (phenylsulfonylamino)cinnamic acid

The title compound was prepared according to the procedure described in step 2 of Example 1 from methyl *trans*4-trifluoromethyl-2-(phenylsulfonylamino)cinnamate (step 2).
¹H-NMR (DMSO-d₆) δ: 10.21 (1H, br s), 7.87 (1H, br d, J=8.4Hz), 7.66 (1H, d, J=16.0Hz), 7.56-7.39 (6H, m), 6.99 (1H, br s), 6.34 (1H, d, J=15.8Hz).

### STEP 4. (E) -3-{4-Trifluoromethyl-2-[(phenylsulfonyl)amino]phenyl}-N-(2-cyanoethyl)-2-propenamide

The title compound was prepared according to the procedure described in step 3 of Example I from *Trans* -4-trifluoromethyl-2-(phenylsulfonylamino)cinnamic acid (step 3).
¹H-NMR (DMSO-d₆) δ: 10.21 (1H, br s), 8.40 (1H, br t, J=5.9Hz), 7.67-7.38 (7H, m), 7.16-7.03 (2H, m), 6.40 (1H, d, J=15.7Hz), 3.40-3.15 (2H, m), 2.62 (2H, t, J=6.3Hz).

### STEP 5. N-(4-Trifluoromethyl-2-{(E)-2-[1-(2-cyanoethyl)-1H-1,2,3,4-tetrazol-5-yl]ethenyl}phenyl)benzenesulfonamide

The title compound was prepared according to the procedure described in step 4 of Example 1 from (E)-3-{4-trifluoromethyl-2-[(phenylsulfonyl)amino]phenyl}-N-(2-cyanoethyl)-2-propenamide (step 4).
¹H-NMR (DMSO-d₆) δ: 10.43 (1H, br s), 8.17 (1H, br d, J=7.91Hz), 8.03 (1H, br d, J=16.2Hz), 7.75-7.42 (7H, m), 7.17 (1H, br s), 4.87 (2H, t, J=6.8Hz), 3.21 (2H, t, J=6.4Hz).

### STEP 6. [6-Trifluoromethyl-3-(1H-1,2,3,4-tetrazol-5-ylmethyl)-1H-indol-2-yl](4-methy-2-pyridinyl)methanone

The title compound was prepared according to the procedure described in step 5 of Example 1 from N-(5-trifluoromethyl-2-{(E)-2-[1-(2-cyanoethyl)-1H-1,2,3,4-tetrazol-5-yl]ethenyl}phenyl)benzenesulfonamide (step 5).
m.p.: 248-252°C.
MS (EI) m/z: 386 (M⁺).
IR (KBr) ν: 1701, 1595, 1508, 1337, 1194, 1119, 1063.
¹H-NMR (DMSO-d₆) δ: 12.79 (1H, br s), 8.70 (1H, d, J=4.94Hz), 8.16 (1H, br s), 7.95-7.91 (2H, m), 7.60 (1H, br d, J=4.6Hz), 7.39 (1H, br d, J=8.6Hz). 4.83 (2H, s), 2.46 (3H, s).

### EXAMPLE 25

### [6-TRIFLUOROMETHYL-3-(1H-1,2,3,4-TETRAZOL-5-YLMETHYL)-1H-INDOL-2-YL](3-ISOOUINOLINYL)METHANONE

The title compound was prepared accocding to the procedure described in step 5 of Example 1 from N-(5-trifluoromethyl-2-{(E)-2-[1-(2-cyanoethyl)-1H-1,2,3,4-tetrazol-5-yl]ethenyl}phenyl)benzenesulfonamide (Example 24, step 5) and 3-bromoacetylisoquinoline hydrobromide (preparation is described in Example 21).
MS (EI) m/z: 422 (M⁺).
IR(KBr) ν: 3273, 1636, 1612, 1529, 1336, 1274, 1209, 1111, 1053, 816.
¹H-NMR (DMSO-d₆) δ: 12.81 (1H, s), 9.55 (1H, s), 8.68 (1H, s), 8.42-8.35 (1H, m), 830-8.22 (2H, m), 8.00-7.87 (3H, m), 7.69-7.61 (1H, m), 4.90 (2H, s).

### EXAMPLE 26

### [5-TRIFLUOROMETHYL-3-(1H-1,2,3,4-TETRAZOL-5-YLMETHYL)-1H-INDOL-2-YL](4-METHYL-2-PYRIDINYL)METHANONE

### STEP 1. Methyl trans-2-amino-5-(trifluoromethyl)cinnamate

The title compound was prepared according to the procedure described in step 1 of Example 24 from 2-bromo-4-(trifluoromethyl)aniline.
¹H-NMR (CDCl₃) δ: 7.77 (1H, d, J=15.8Hz), 7.61 (1H, s), 7.39 (1H, d, J=8.4Hz), 6.74 (1H, d, J=8.4Hz), 6.41 (1H, dd, J=15.8, 1.5Hz), 4.29 (2H, m), 3.82 (3H, m).

### STEP 2. Methyl trans-2-phenylsulfonylamino-5-(trifluoromethyl)cinnamate

The title compound was prepared according to the procedure described in step 1 of Example 1 from methyl *trans*-2-amino-5-(trifluoromethyl)cinnamate (step 1). ¹H-NMR (CDCl₃) δ: 7.79-7.76 (2H, m), 7.66 (1H, m), 7.60-7.44 (6H, m), 7.06 (1H, br s), 6.26 (1H, d, J=15.8Hz), 3.81 (3H, s).

### STEP 3. trans-5-Trifluoromethyl-2-(phenylsulfonylamino)cinnamic acid

The title compound was prepared according to the procedure described in step 2 of Example 1 from methyl *trans*5-trifluoromethyl-2-(phenylsulfonylamino)cinnamate (step 2).
¹H-NMR (DMSO-d₆) δ: 10.36 (1H, br s), 7.95 (1H, br s), 7.65 (1H, d, J=16.0Hz), 7.59-7.50 (4H, m), 7.46-7.40 (2H, m), 7.07 (1H, d, J=8.2Hz), 6.42 (1H, d, J=16.0Hz).

### STEP 4. (E)-3-{5-Trifluoromethyl-2-[(phenylsulfonyl)amino]phenyl}-N-(2-cyanoethyl)-2-propenamide

The title compound was prepared according to the procedure described in step 3 of Example 1 from *trans*-4-trifluoromethyl-2-(phenylsulfonylamino)cinnamic acid (step 3).
¹H-NMR (DMSO-d₆) δ: 10.38 (1H, br s), 8.30 (1H, br t, J=5.9Hz), 7.71 (1H, br s), 7.61-7.39 (7H, m), 7.10 (1H, d, J=8.4Hz), 6.45 (1H, d, J=15.8Hz), 3.40-3.15 (2H, m), 2.62 (2H, t, J=6.3Hz).

### STEP 5. N-5-Trifluoromethyl-2-{(E)-2-[1-(2-cyanoethyl)-1H-1,2,3,4-tetrazol-5-yl]ethenyl}phenyl)benzenesulfonamide

The title compound was prepared according to the procedure described in step 4 of Example 1 from (E)-3-{5-trifluoromethyl-2-[(phenylsulfonyl)amino]phenyl}-N-(2-cyanoethyl)-2-propenamide (step 4).
¹H-NMR (DMSO-d₆) δ: 7.92-7.79 (4H, m), 7.58-7.42 (5H, m), 6.92 (1H, d, J=16.0Hz), 6.42 (1H, br s), 4.74 (2H, t, J=6.6Hz), 3.13 (2H, t, J=6.6Hz).

### STEP 6. [5-Trifluoromethyl-3-(1H-1,2,3,4-tetrazol-5-ylmethyl)-1H-indol-2-yl](4-methy-2-pyridinyl)methanone

The title compound was prepared according to the procedure described in step 5 of Example 1 from N-(4-trifluoromethyl-2-{(E)-2-[1-(2-cyanoethyl)-lH-1,2,3,4-tetrazol-5-yl]ethenyl}phenyl)benzenesulfonamide (step 5).
m.p.: 263-265°C.
MS (EI) m/z: 386 (M⁺).
IR (KBr) ν: 1647, 1597, 1537, 1450, 1408, 1312, 1273, 1207, 1099.
¹H-NMR (DMSO-d₆) δ: 12.68 (1H, br s), 8.69 (1H, d, J=4.9Hz), 8.21 (1H, br s), 7.93-7.89 (2H, m), 7.63 (1H, dd, J=1.5, 8.9Hz), 7.59 (1H, br d, J=4.9Hz), 4.84 (2H, s), 2.45 (3H, s).

### EXAMPLE 27

### [5-FLUORO-3-(1H-1,2,3,4-TETRAZOL-5-YLMETHYL)-1H-INDOL-2-YL](4-METHYL-2-PYRIDINYL)METHANONE

### STEP 1.Methyl trans-5-fluoro-2-nitrocinnamate

The title compound was prepared according to the procedure described in step 1 of Example 23 from 5-fluoro-2-nitrobenzaldehyde.
¹H-NMR (CDCl₃) δ: 8.17-8.10 (2H, m), 7.32-7.19 (2H, m), 6.36 (1H, d, J=15.8Hz), 3.84 (3H, s).

### STEP 2. Methyl trans-5-fluoro-2-aminocinnamate

The title compound was prepared according to the procedure described in step 2 of Example 23 from methyl *trans*-5-fluoro-2-nitrocinnamate (step 1).
¹H-NMR (CDCl₃) δ: 7.77 (1H, dd, J=15.8, 1.5Hz), 7.10-7.06 (1H, m), 6.94-6.87 (1H, m), 6.68-6.63 (1H, m), 6.33 (1H, d, J=15.8Hz), 3.85 (2H, m), 3.81 (3H, s).

### STEP 3. Methyl trans-5-fluoro-2-(phenylsulfonylamino)cinnamate

The title compound was prepared according to the procedure described in step 1 of Example 1 from methyl *trans*-5-fluoro-2-aminocinnamate (step 2).
H-NMR (CDCl₃) δ: 7.68-7.65 (2H, m), 7.55-7.49 (2H, m), 7.44-7.33 (3H, m), 7.18-7.13 (1H, m), 7.10-7.04 (1H, m), 6.10 (1H, d, J=15.8Hz), 3.78 (3H, s).

### STEP 4. trans-5-Fluoro-2-(phenylsulfonylamino)cinnamic acid

The title compound was prepared according to the procedure described in step 2 of Example 1 from methyl *trans*5-fluoro-2-(phenylsulfonylamino) cinnamate (step 3).
¹H-NMR (DMSO-d₆) δ: 7.72-7.48 (7H, m), 7.15 (1H, dt, J=3.0, 8.4Hz), 6.88 (1H, dd, J=5.4.8.9Hz), 6.40 (1H, d, J=16.0Hz).

### STEP 5. (E)-3-{5-Fluoro-2-[(phenylsulfonyl)amino]phenyl}-N-(2-cyanoethyl)-2-propenamide

The title compound was prepared according to the procedure described in step 3 of Example 1 from *trans* 4-fluoro-2-(phenylsulfonylamino)cinnamic acid (step 4).
¹H-NMR (DMSO-d₆) δ: 9,98 (1H, br s), 8.37 (1H, br t, J=5.8Hz), 7.64-7.48 (6H, m), 7.38 (1H, dd, J=2.8, 9.9Hz), 7.15 (1H, dt, J=3.0, 8.7Hz), 6.91 (1H, dd, J=5.6, 8.9Hz), 6.41 (1H, d, J=15.7Hz), 3.42 (2H, dt, J=6.1, 6.1Hz), 2.73 (2H, t, J=6.4Hz).

### STEP 6. N-(5-Fluoro-2-{(E)-2-[1-(2-cyanoethyl)-1H-1,2,3,4-tetrazol-5-yl]ethenyl}phenyl)benzenesulfonamide

The title compound was prepared according to the procedure described in step 4 of Example 1 from (E)-3-{5-fluoro-2-[(phenylsulfonyl)amino]phenyl}-N-(2-cyanoethyl)-2-propenamide (step 5).
H-NMR (CDCl₃) δ: 7.84 (1H, d, J=16.3Hz), 7.72-7.67 (2H, m), 7.54-7.29 (5H, m), 7.09-6.96 (2H, m), 6.89 (1H, d, J=16.3Hz), 4.75 (2H, t, J=6.59Hz), 3.15 (2H, t, J=6.59Hz).

### STEP 7. [5-Fluoro-3-(1H-1,2,3,4-tetrazol-5-ylmethyl)-1H-indol-2-yl](4-methy-2-pyridinyl)methanone

The title compound was prepared according to the procedure described in step 5 of Example 1 from N-(4-fluoro-2-{(E)-2-[1-(2-cyanoethyl)-1H-1,2,3,4-tetrazol-5-yl]ethenyl}phenyl)benzenesulfonamide (step 6).
m.p.: 253-257°C.
MS (EI) m/z: 336 (M⁺).
¹H-NMR (DMSO-d₆) δ: 12.44 (1H, br s), 8.69 (1H, d, J=4.9Hz), 7.91 (1H, br s), 7.74 (1H, dd, J=4.6, 9.1Hz), 7.57 (1H, br d, J=5.1Hz), 7.49 (1H, dd, J=2.5, 9.9Hz), 7.24 (1H, dt, J=2.3, 9.2Hz), 4.74(2H, s), 2.45 (3H, s).

### EXAMPLE 28

### [6-FLUORO-3-(1H-1,2,3,4-TETRAZOL-5-YLMETHYL)-1H-INDOL-2-YL](4-METHYL-2-PYRIDINYL)METHANONE

### STEP 1. Methyl trans-(4-fluoro-2-nitro)cinnamate

The title compound was prepared according to the procedure described in step 1 of Example 24 from 3-fluoro-6-iodonitrobenzene and methyl acrylate .
¹H-NMR (CDCl₃) δ: 8.67 (1H, d, 15.8Hz), 7.78 (1H, dd, 8.07Hz, 2.65Hz), 7.68-7.63 (2H, m), 6.34 (1H, d, 15.8Hz), 3.84 (3H, s).

### STEP 2. Methyl trans-(2-amino-4-fluoro)cinnamate

The title compound was prepared according to the procedure described in step 2 of Example 23 from methyl *trans-*(4-fluoro-2-nitro)cinnamate (step 1).
¹H-NMR (CDCl₃) δ: 7.75 (1H, d, 15.8Hz), 7.37-7.31 (1H, m), 6.50-6.37 (2H, m), 6.32-6.26 (1H, m), 4.13 (2H, br s), 3.80 (3H, s).

### STEP 3. Methyl trans-4-Fluoro-2-(p-toluenesulfonylamino)cinnamate

The title compound was prepared according to the procedure described in step 1 of Example 1 from methyl *trans-*(2-amino-4-fluoro)cinnamate (step 2).
¹H-NMR (CDCl₃) δ: 7.64 (1H, d, 8.40Hz), 7.46-7.19 (4H, m), 6.94-6.87 (1H, m), 6.77 (1H, s), 6.16-6.10 (1H, m), 3.79 (3H, s), 2.38 (3H, s) .

### STEP 4. trans-4-Fluoro-2-[[(4-methylphenyl)sulfonyl]amino]cinnamic acid

The title compound was prepared according to the procedure described in step 2 of Example 1 from methyl *trans*-4-Fluoro-2-(p-toluenesulfonylamino)cinnamate (step 3).
¹H-NMR (DMSO-d₆) δ: 12.35 (1H, br s), 10.21 (1H, s), 7.84-7.76 (1H, m), 7.66 (1H, d, J=16.0 Hz), 7.53 (2H, d, J=8.2 Hz), 7.33 (2H, d, J=8.2 Hz), 7.18-7.07 (1H, m), 6.76 (1H, dd, J=2.6, 8.9 Hz), 6.25 (1H, d, J=16.0 Hz), 2.35 (3H, s).

### STEP 5. (E)-3-{4-Fluoro-2-[[(4-methylphenyl)sulfonyl]amino]phenyl}-N-(2-cyanoethyl)-2-propenamide

The title compound was prepared according to the procedure described in step 3 of Example 1 from *trans*-4-fluoro-2-[[(4-methylphenyl)sulfonyl]amino]cinnamic acid (step 4).
¹H-NMR (CDCl₃) δ: 8.01 (1H, br s), 7.65 (2H, d, J=8.2 Hz), 7.60 (1H, d, J=15.5 Hz), 7.38 (1H, dd, J=6.2, 8.9 Hz), 7.25-7.16 (3H, m), 6.92-6.83 (1H, m), 6.49 (1H, br t, J=6.3 Hz), 6.17 (1H, d, J=15.5 Hz), 3.70-3.60 (2H, m), 2.70 (2H, t, J=6.3 Hz), 2.37 (3H, S).

### STEP 6. N-(5-Methyl-2-{(E)-2-[1-(2-cyanoethyl)-1H-1,2,3,4-tetrazol-5- yl]ethenyl}phenyl)-4-methylbenzenesulfonamide

The title compound was prepared according to the procedure described in step 4 of Example 1 from (E)-3-{4-fluoro-2-[[(4-methylphenyl)sulfonyl]amino]phenyl}-N-(2-cyanoethyl)-2-propenamide (step 5).
¹H-NMR (DMSO-d₆) δ: 7.83 (1H, d, J=16.1 Hz), 7.68 (2H, d, J=8.2 Hz), 7.60-7.52 (1H, m), 7.25 (2H, d, J=8.2 Hz), 7.02-6.90 (2H, m), 6.83 (1H, d, J=16.1 Hz), 4.72 (2H, t, J=6.6 Hz), 3.11 (2H, t, J=6.6 Hz), 2.37 (3H, s).

### STEP 7. [6-Fluoro-3-(1H-1,2,3,4-tetrazol-5-ylmethyl)-1H-indol-2-yl](4-methyl-2-pyridinyl)methanone

The title compound was prepared according to the procedure described in step 5 of Example 1 from N-(5-methyl-2-{(E)-2-[1-(2-cyanoethyl)-1H-1,2,3,4-tetrazol-5-yl]ethenyl}phenyl)-4-methylbenzenesulfonamide (step 6) and 2-bromoacetyl-4-methylpyridine hydrobromide (preparation is described in step 5 of Example 1).
MS (ESI) m/z: 335 (MH⁻).
IR (KBr) ν: 3275, 2868, 1641, 1597, 1533, 1281, 1213, 1130, 800.
¹H-NMR (DMSO-d₆) δ: 12.44 (1H, s), 8.69 (1H, d, J=4.9 Hz), 7.94-7.90 (1H, m), 7.74 (1H, dd, J=5.6, 8.9 Hz), 7.60-7.55 (1H, m), 7.48 (1H, dd, J=2.0, 10.2 Hz), 7.06-6.95 (1H, m), 4.79 (2H, s), 2.45 (3H, s).

### EXAMPLE 29

### [6-METHYL-3-(1H-1,2,3,4-TETRAZOL-5-YLMETHYL)-1H-INDOL-2-YL](4-METHYL-2-PYRIDINYL)METHANONE

### STEP 1. Methyl trans-2-amino-4-methylcinnamate

The title compound was prepared according to the procedure described in step 2 of Example 23 from methyl *trans*-4-methyl-2-nitrocinnamate.
¹H-NMR (CDCl₃) δ: 7.81 (1H, d, J=15.8Hz), 7.30-7.26 (1H, m), 6.60-6.57 (1H, m), 6.52 (1H, m), 6.31 (1H, d, J=15.8Hz), 3.92 (2H, br s), 3.79 (3H, s), 2.26 (2H, s).

### STEP 2. Methyl trans-4-methyl-2-(phenylsulfonylamino)cinnamate

The title compound was prepared according to the procedure described in step 1 of Example 1 from methyl *trans*-2-amino-4-methylcinnamate (step 1).
¹H-NMR (CDCl₃) δ: 7.72-7.68 (2H, m), 7.55-7.49 (1H, m), 7.44-7.34 (4H, m), 7.26-7.22 (1H, m), 7.07-7.04 (1H, m), 6.62 (1H, br s), 6.12 (1H, d, J=15.8Hz), 3.76 (3H, s), 2.34 (3H, s).

### STEP 3. trans-4-methyl-2-(phenylsulfonylamino)cinnamic acid

The title compound was prepared according to the procedure described in step 2 of Example 1 from methyl *trans*-4-methyl-2-(phenylsulfonylamino)cinnamate (step 2).
¹H-NMR (DMSO-d₆) δ: 12.25 (1H, br s), 9.96 (1H, br s), 7.73 (1H, d, J=16.0 Hz), 7.67-7.47 (6H, m), 7.08 (1H, d, J=8.2 Hz), 6.70 (1H, s), 6.25 (1H, d, J=16.0 Hz), 2.18 (3H, s).

### STEP 4. (E)-3-{4-methyl-2-[(phenylsulfonyl)amino]phenyl}-N-(2-cyanoethyl)-2-propenamide

The title compound was prepared according to the procedure described in step 3 of Example 1 from *trans*-4-methyl-2-(phenylsulfonylamino)cinnamic acid (step 3).
¹H-NMR (CDCl₃) δ: 8.67 (1H, s), 7.74-6.80 (9H, m), 6.05 (1H, d, J=15.5 Hz), 3.66-3.54 (2H, m), 2.73-2.66 (2H, m), 2.29 (3H, S).

### STEP 5. N-(5-methyl-2-{(E)-2-[1-(2-cyanoethyl)-1H-1,2,3,4-tetrazol-5-yl]ethenyl}phenyl)benzenesulfonamide

The title compound was prepared according to the procedure described in step 4 of Example 1 from (E)-3-{4-methyl-2-[(phenylsulfonyl)amino]phenyl}-N-(2-cyanoethyl)-2-propenamide (step 4).
¹H-NMR (DMSO-d₆) δ: 10.06 (1H, s), 7.95 (1H, d, J=16.0 Hz), 7.86 (1H, d, J=8.1 Hz), 7.74-7.40 (6H, m), 7.20 (1H, d, J=16.0 Hz), 7.17 (1H, d, J=7.7 Hz), 4.83 (2H, t, J=6.4 Hz), 3.19 (2H, t, J=6.4 Hz), 2.22 (3H, s).

### STEP 6. [6-Methyl-3-(1H-1,2,3,4-tetrazol-5-ylmethyl)-1H-indol-2-yl](4-methyl-2-pyridinyl)methanone

The title compound was prepared according to the procedure described in step 5 of Example 1 from N-(5-methyl-2-{(E)-2-[1-(2-cyanoethyl)-1H-1,2,3,4-tetrazol-5-yl]ethenyl}phenyl)benzenesulfonamide (step 5) and 2-bromoacetyl-4-methylpyridine hydrobromide (preparation is described in step 5 of example 1).
MS (EI) m/z : 332 (M⁺).
IR (KBr) ν: 3298, 2858, 1641, 1599, 1531, 1406, 1281, 1209, 1138, 1047, 799.
¹H-NMR (DMSO-d₆) δ: 12.20 (1H, s), 8.67 (1H, d, J=5.1 Hz), 7.90 (1H, s), 7.58-7.45 (3H, m), 6.95 (1H, d, J=8.4 Hz), 4.75 (2H, s), 2.45 (3H, s), 2.43 (3H, s).

The chemical structures of the compounds prepared in the Examples 1 to 29 are summarized in the following tables.

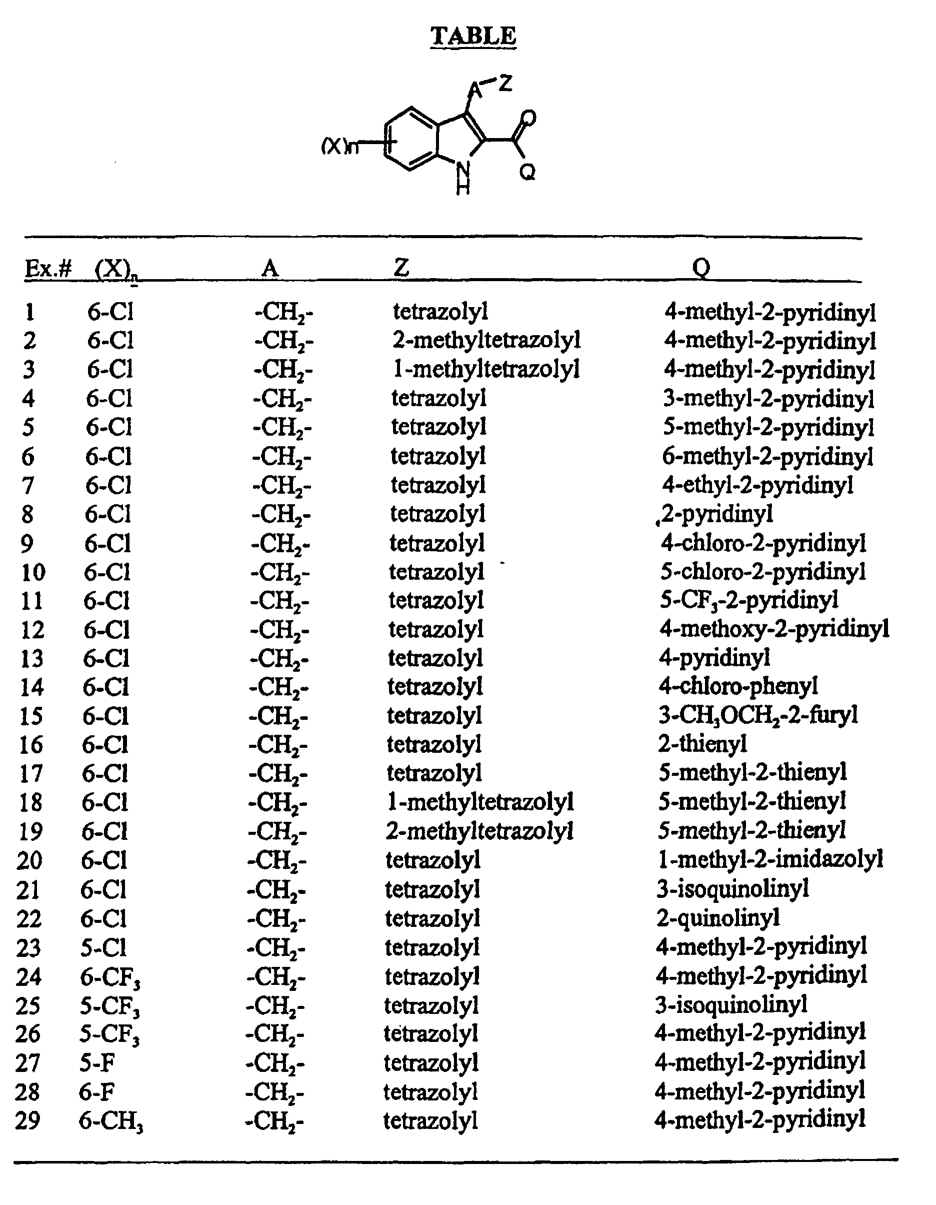

## Claims

1. A compound of the following formula: 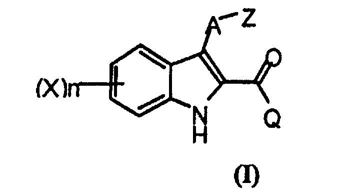 or the pharmaceutically acceptable salts thereof wherein
**Z** is tetrazolyl optionally substituted with C₁₋₄ alkyl or halosubstituted C₁₋₄ alkyl;
**A** is C₁₋₆ alkylene;
**Q** is a cyclic group selected from:
(a) phenyl;
(b) a partially saturated, fully saturated or fully unsaturated five to six membered monocyclic group having one to four heteroatoms selected independently from N, O and S; and
(c) a bicyclic group consisting of two fused partially saturated, fully saturated or fully unsaturated five to eight membered rings independently and optionally having one to four heteroatoms selected independently from N, O and S,
said cyclic groups (a), (b) and (c) being independently and optionally substituted with one to five substituents independently selected from halogen, C₁₋₄ alkyl, halosubstituted C₁₋₄ alkyl, OH, C₁₋₄ alkoxy, halosubstituted C₁₋₄ alkoxy, C₁₋₄ alkylthio, NO₂, NH₂, mono- or di-(C₁₋₄ alkyl)amino, CN, hydroxy-C₁₋₄ alkyl and C₁₋₄ alkoxy-C₁₋₄ alkyl;
**X** is independently selected from halogen, C₁₋₄ alkyl, halosubstituted C₁₋₄ alkyl, OH, C₁₋₄ alkoxy, halosubstituted C₁₋₄ alkoxy, C₁₋₄ alkylthio, NO₂, NH₂ and mono- or di-(C₁₋₄ alkyl)amino and CN; and
**n** is 0, 1, 2, 3 or 4;

2. A compound according to claim 1, wherein
**Z** is tetrazolyl optionally substituted with C₁₋₃ alkyl or halosubstituted C₁₋₃ alkyl;
**A** is C₁₋₅ alkylene;
**Q** is a cyclic group selected from:
(a) phenyl;
(b) a partially saturated, fully saturated or fully unsaturated five to six membered monocyclic group having one to three heteroatoms selected independently from N, O and S; and
(c) a bicyclic group consisting of two fused partially saturated, fully saturated or fully unsaturated five or six membered rings independently and optionally having one to three heteroatoms selected independently from N, O and S,
said cyclic groups (a), (b) and (c) being independently and optionally substituted with one, two or three substituents independently selected from halogen, C₁₋₄ alkyl, halosubstituted C₁₋₄ alkyl, C₁₋₄ alkoxy, halosubstituted C₁₋₄ alkoxy and C₁₋₄ alkoxy-C₁₋ ₄ alkyl;
**X** is independently selected from halogen, C₁₋₄ alkyl, halosubstituted C₁₋₄ alkyl, OH and C₁₋₄ alkoxy; and
**n** is 0, 1, 2 or 3.

3. A compound according to claim 1, wherein
**Z** is tetrazolyl;
**A** is C₁₋₄ alkylene;
**Q** is selected from the following groups:
(a) phenyl;
(b) a five membered monocyclic gourp selected from furyl, thienyl, pyrrolyl, pyrrolinyl, pyrrolidinyl, dioxolanyl, oxazolyl, thiazolyl, imidazolyl, imidazolinyl, imidazolidinyl, pyrazolyl, pyrazolinyl, pyrazolidinyl, isoxazolyl, isothiazolyl, dithiolyl, oxathiolyl, oxadiazolyl, thiadiazolyl, oxatriazolyl, dioxazolyl, oxathiazolyl and oxathiolyl, and a six membered monocyclic group selected from pyranyl, pyridinyl, piperidinyl, dioxinyl, dioxanyl, morpholinyl, dithianyl, thiomorpholinyl, pyridazinyl, pyrimidinyl, pyrazinyl, piperazinyl, triazinyl, triazinyl, trithianyl, oxazinyl, oxathiazinyl, isoxazinyl and oxadiazinyl.; and
(c) a bicyclic group selected from indolyl, isoindolyl, indolinyl, benzofuryl, dihydrobenzofuranyl, isobenzofuryl, benzothienyl, indazolyl, indoxazinyl, benzoxazolyl, anthranilyl, benzimidazolyl, benzthiazolyl, purinyl, quinolizinyl, quinolinyl, isoquinolinyl, tetrahydroquinolinyl, tetrahydroisoquinolinyl, cinnolinyl, phthalazinyl, quinazolinyl, quinoxalinyl, naphthyridinyl, pteridinyl, indenyl, isoindenyl, naphtyl, tetralinyl, decalinyl, benzopyranyl, pyridopyridinyl, benzoxazinyl, dihydrobenzodioxinyl, benzodioxolyl, indanyl, cyclopentapyridinyl, pyranopyrrolyl, chromanyl and isochromanyl;
said cyclic groups (a), (b) and (c) being independently and optionally substituted with one, two or three substituents independently selected from halogen, C₁₋₃ alkyl, halosubstituted C₁₋₃ alkyl, C₁₋₃ alkoxy, halosubstituted C₁₋₃ alkoxy and C₁₋₃ alkoxy-C₁₋₃ alkyl;
**X** is independently selected from halogen, C₁₋₄ alkyl and halosubstituted C₁₋₄ alkyl;
**n** is 0, 1 or 2.

4. A compound according to claim 3, wherein **Z** is tetrazolyl; **A** is methylene; **Q** is a cyclic group selected from pyridinyl, phenyl, furyl, thienyl, imidazolyl, quinolinyl and isoquinolinyl, and the cyclic group being optionally substituted with methyl, ethyl, chloro, trifluoromethyl, methoxy or methoxymethy I: **X** is chloro, fluoro, methyl or trifluoromethyl; and **n** is 0 or 1.

5. A compound according to claim 4, wherein **Z** is 1,2,3,4-tetrazolyl; **Q** is 2- or 4-pyridinyl, 3-, 4-, 5- or 6-methyl-2-pyridinyl, 4-ethyl-2-pyridinyl, 4-or 5-chloro-2-pyridinyl, 5-trifuloromethyl-2-pyridinyl, 4-methoxy-2-pyridinyl, 4-chlorophenyl, 3-methoxymethyl-2-furyl, 2-thienyl, 5-methyl-2-thienyl, 1-methyl-2 -imidazolyl, 3-isoquinolinyl or 2-quinolinyl; and **X** is 6-chloro, 5-chloro, 6-trifluoromethyl, 5-trifluoromethyl, 5-fluoro, 6-fluoro or 6-methyl.

6. A compound according to claim 1 selected from
[6-chloro-3-(1H-1,2,3,4-tetrazol-5-ylmethyl)-1H-indol-2-yl](4-methyl-2-pyridinyl)-methanone;
{6-chloro-3-[(2-methyl-2H-1,2,3,4-tetrazol-5-yl)methyl]-1H-indol-2-yl}(4-methyl-2-pyridinyl)methanone;
6-chloro-3-[(1-methyl-1H-1,2,3,4-tetrazol-5-yl)methyl]-1H-indol-2-yl}(4-methyl-2-pyridinyl)methanone;
[6-chloro-3-(1H-1,2,3,4-tetrazol-5-ylmethyl)-1H-indol-2-yl](3-methyl-2-pyridinyl)-methanone;
[6-chloro-3-(1H-1,2,3,4-tetrazol-5-ylmethyl)-1H-indol-2-yl](5-methyl-2-pyridinyl)-methanone;
[6-chloro-3-(1H-1,2,3,4-tetrazol-5-ylmethyl)-1H-indol-2-yl](6-methyl-2-pyridinyl)-methanone;
[6-chloro-3-(1H-1,2,3,4-tetrazol-5-ylmethyl)-1H-indol-2-yl](4-ethyl-2-pyridinyl)-methanone;
[6-chloro-3-(1H-1,2,3,4-tetrazol-5-ylmethyl)-1H-indol-2-yl](2-pyridinyl)methanone;
[6-chloro-3-(1H-1,2,3,4-tetrazol-5-ylmethyl)-1H-indol-2-yl](4-chloro-2-pyridinyl)-methanone;
[6-chloro-3-(1H-1,2,3,4-tetrazol-5-ylmethyl)-1H-indol-2-yl](5-chloro-2-pyridinyl)-methanone;
[6-chloro-3-(1H-1,2,3,4-tetrazol-5-ylmethyl)-1H-indol-2-yl](5-trifluoromethyl-2-pyridinyl)methanone;
[6-chloro-3-(1H-1,2,3,4-tetrazol-5-ylmethyl)-1H-indol-2-yl](4-methoxy-2-pyridinyl)-methanone;
[6-chloro-3-(1H-1,2,3,4-tetrazol-5-ylmethyl)-1H-indol-2-yl](4-pyridinyl)methanone;
[6-chloro-3-(1H-1,2,3,4-tetrazol-5-ylmethyl)-1H-indol-2-yl](4-chlorophenyl)-methanone;
[6-chloro-3-(1H-1,2,3,4-tetrazol-5-ylmethyl)-1H-indol-2-yl](3-methoxymethyl-2-furyl)methanone;
[6-chloro-3-(1H-1,2,3,4-tetrazol-5-ylmethyl)-1H-indol-2-yl](2-thienyl)methanone;
[6-chloro-3-(1H-1,2,3,4-tetrazol-5-ylmethyl)-1H-indol-2-yl](5-methyl-2-thienyl)-methanone;
{6-chloro-3-[(1-methyl-1H-1,2,3,4-tetrazol-5-yl)methyl]-1H-indol-2-yl}(5-methyl-2-thienyl)methanone;
{6-chloro-3-[(2-methyl-2H-1,2,3,4-tetrazol-5-yl)methyl]-1H-indol-2-yl}(5-methyl-2-thienyl)methanone;
[6-chloro-3-(1H-1,2,3,4-tetrazol-5-ylmethyl)-1H-indol-2-yl](1-methyl-2-imidazolyl)-methanone;
[6-chloro-3-(1H-1,2,3,4-tetrazol-5-ylmethyl)-1H-indol-2-yl](3-isoquinolinyl)-methanone;
[6-chloro-3-(1H-1,2,3,4-tetrazol-5-ylmethyl)-1H-indol-2-yl](2-quinolinyl)methanone;
[5-chloro-3-(1H-1,2,3,4-tetrazol-5-ylmethyl)-1H-indol-2-yl](4-methyl-2-pyridinyl)-methanone;
[6-trifluoromethyl-3-(1H-1,2,3,4-tetrazol-5-ylmethyl)-1H-indol-2-yl](4-methyl-2-pyridinyl)methanone;
[6-trifluoromethyl-3-(1H-1,2,3,4-tetrazol-5-ylmethyl)-1H-indol-2-yl](3-isoquinolinyl) methanone;
[5-trifluoromethyl-3-(1H-1,2,3,4-tetrazol-5-ylmethyl)-1H-indol-2-yl](4-methyl-2-pyridinyl)methanone;
[5-fluoro-3-(1H-1,2,3,4-tetrazol-5-ylmethyl)-1H-indol-2-yl](4-methyl-2-pyridinyl)-methanone;
[6-fluoro-3-(1H-1,2,3,4-tetrazol-5-ylmethyl)-1H-indol-2-yl](4-methyl-2-pyridinyl)-methanone;
[6-methyl-3-(1H-1,2,3,4-tetrazol-5-ylmethyl)-1H-indol-2-yl](4-methyl-2-pyridinyl)-methanone,
and the salts thereof.

7. A compound according to claim 6, selected from:
[6-chloro-3-(1H-1,2,3,4-tetrazol-5-ylmethyl)-1H-indol-2-yl](4-methyl-2-pyridinyl)-methanone;
{6-chloro-3-[(2-methyl-2H-1,2,3,4-tetrazol-5-yl)methyl]-1H-indol-2-yl}(4-methyl-2-pyridinyl)methanone;
6-chloro-3-[(1-methyl-1H-1,2,3,4-tetrazol-5-yl)methyl]-1H-indol-2-yl}(4-methyl-2-pyridinyl)methanone;
[6-chloro-3-(1H-1,2,3,4-tetrazol-5-ylmethyl)-1H-indol-2-yl](5-methyl-2-pyridinyl)-methanone;
[6-chloro-3-(1H-1,2,3,4-tetrazol-5-ylmethyl)-1H-indol-2-yl](4-ethyl-2-pyridinyl)-methanone;
[6-chloro-3-(1H-1,2,3,4-tetrazol-5-ylmethyl)-1H-indol-2-yl](4-chloro-2-pyridinyl)-methanone;
[6-chloro-3-(1H-1,2,3,4-tetrazol-5-ylmethyl)-1H-indol-2-yl](5-chloro-2-pyridinyl)-methanone;
[6-chloro-3-(1H-1,2,3,4-tetrazol-5-ylmethyl)-1H-indol-2-yl](4-methoxy-2-pyridinyl)-methanone;
[6-chloro-3-(1H-1,2,3,4-tetrazol-5-ylmethyl)-1H-indol-2-yl](5-methyl-2-thienyl)-methanone;
{6-chloro-3-[(1-methyl-1H-1,2,3,4-tetrazol-5-yl)methyl]-1H-indol-2-yl}(5-methyl-2-thienyl)methanone;
{6-chloro-3-[(2-methyl-2H-1,2,3,4-tetrazol-5-yl)methyl]-1H-indol-2-yl}(5-methyl-2-thienyl)methanone;
[6-chloro-3-(1H-1,2,3,4-tetrazol-5-ylmethyl)-1H-indol-2-yl](3-isoquinolinyl)-methanone;
[6-trifluoromethyl-3-(1H-1,2,3,4-tetrazol-5-ylmethyl)-1H-indol-2-yl](3-isoquinolinyl) methanone;
[5-trifluoromethyl-3-(1H-1,2,3,4-tetrazol-5-ylmethyl)-1H-indol-2-yl](4-methyl-2-pyridinyl)methanone;
[5-fluoro-3-(1H-1,2,3,4-tetrazol-5-ylmethyl)-1H-indol-2-yl](4-methyl-2-pyridinyl)-methanone;
[6-fluoro-3-(1H-1,2,3,4-tetrazol-5-ylmethyl)-1H-indol-2-yl](4-methyl-2-pyridinyl)-methanone;
and the salts thereof.

8. A pharmaceutical composition useful for the treatment of a medical condition in which prostaglandins are implicated as pathogens, which comprises a compound of the formula (I) of claim 1, and a pharmaceutically inert carrier.

9. Use of a compound according to Claim 1 for the manufacture of a medicament for the treatment of a medical condition in a mammalian subject in which prostaglandins are implicated as pathogens.

## Patentansprüche

1. Verbindung der nachstehenden Formel: 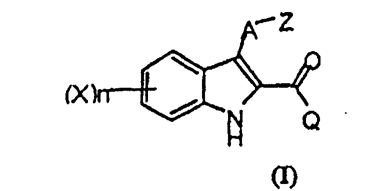 oder die pharmazeutisch verträglichen Salze davon, worin
Z Tetrazolyl, gegebenenfalls substituiert mit C₁₋₄-Alkyl oder Halogen-substituiertem C₁₋₄-Alkyl, darstellt;
A C₁₋₆-Alkylen darstellt;
Q eine cyclische Gruppe darstellt, ausgewählt aus:
(a) Phenyl;
(b) einer teilweise gesättigten, vollständig gesättigten oder vollständig ungesättigten fünf- bis sechsgliedrigen monocyclischen Gruppe mit ein bis vier Heteroatomen, unabhängig ausgewählt aus N, O und S; und
(c) einer bicyclischen Gruppe, bestehend aus zwei kondensierten, teilweise gesättigten, vollständig gesättigten oder vollständig ungesättigten fünf- bis achtgliedrigen Ringen, unabhängig und gegebenenfalls mit ein bis vier Heteroatomen, unabhängig ausgewählt aus N, O und S,
wobei die cyclischen Gruppen (a), (b) und (c) unabhängig und gegebenenfalls mit ein bis fünf Substituenten, unabhängig ausgewählt aus Halogen, C₁₋₄-Alkyl, Halogen-substituiertem C₁₋₄-Alkyl, OH, C₁₋₄-Alkoxy, Halogen-substituiertem C₁₋₄-Alkoxy, C₁₋₄-Alkylthio, NO₂, NH₂, Mono- oder Di-(C₁₋₄-alkyl)amino, CN, Hydroxy-C₁₋₄-alkyl und C₁₋₄-Alkoxy-C₁₋₄-alkyl substituiert sind;
X unabhängig ausgewählt ist aus Halogen, C₁₋₄-Alkyl, Halogen-substituiertem C₁₋₄-Alkyl, OH, C₁₋₄-Alkoxy, Halogen-substituiertem C₁₋₄-Alkoxy, C₁₋₄-Alkylthio, NO₂, NH₂ und Monooder Di-(C₁₋₄-alkyl)amino und CN; und
n 0, 1, 2, 3 oder 4 ist.

2. Verbindung nach Anspruch 1, worin
Z Tetrazolyl, gegebenenfalls substituiert mit C₁₋₃-Alkyl oder Halogen-substituiertem C₁₋₃-Alkyl, darstellt;
A C₁₋₅-Alkylen darstellt;
Q eine cyclische Gruppe darstellt, ausgewählt aus:
(a) Phenyl;
(b) einer teilweise gesättigten, vollständig gesättigten oder vollständig ungesättigten fünf- bis sechsgliedrigen monocyclischen Gruppe mit ein bis drei Heteroatomen, unabhängig ausgewählt aus N, O und S; und
(c) einer bicyclischen Gruppe, bestehend aus zwei kondensierten, teilweise gesättigten, vollständig gesättigten oder vollständig ungesättigten fünf- oder sechsgliedrigen Ringen, unabhängig und gegebenenfalls mit ein bis drei Heteroatomen, unabhängig ausgewählt aus N, O und S,
wobei die cyclischen Gruppen (a), (b) und (c) unabhängig und gegebenenfalls mit ein, zwei oder drei Substituenten, unabhängig ausgewählt aus Halogen, C₁₋₄-Alkyl, Halogen-substituiertem C₁₋₄-Alkyl, C₁₋₄-Alkoxy, Halogen-substituiertem C₁₋₄-Alkoxy und C₁₋₄-Alkoxy-C₁₋₄-alkyl, substituiert sind;
X unabhängig ausgewählt ist aus Halogen, C₁₋₄-Alkyl, Halogen-substituiertem C₁₋₄-Alkyl, OH und C₁₋₄-Alkoxy; und
n 0, 1, 2 oder 3 ist.

3. Verbindung nach Anspruch 1, worin
Z Tetrazolyl darstellt;
A C₁₋₄-Alkylen darstellt;
Q ausgewählt ist aus den nachstehenden Gruppen:
(a) Phenyl;
(b) einer fünfgliedrigen monocyclischen Gruppe, ausgewählt aus Furyl, Thienyl, Pyrrolyl, Pyrrolinyl, Pyrrolidinyl, Dioxolanyl, Oxazolyl, Thiazolyl, Imidazolyl, Imidazolinyl, Imidazolidinyl, Pyrazolyl, Pyrazolinyl, Pyrazolidinyl, Isoxazolyl, Isothiazolyl, Dithiolyl, Oxathiolyl, Oxadiazolyl, Thiadiazolyl, Oxatriazolyl, Dioxazolyl, Oxathiazolyl und Oxathiolyl, und einer sechsgliedrigen monocyclischen Gruppe, ausgewählt aus Pyranyl, Pyridinyl, Piperidinyl, Dioxinyl, Dioxanyl, Morpholinyl, Dithianyl, Thiomorpholinyl, Pyridazinyl, Pyrimidinyl, Pyrazinyl, Piperazinyl, Triazinyl, Triazinyl, Trithianyl, Oxazinyl, Oxathiazinyl, Isoxazinyl und Oxadiazinyl; und
(c) einer bicyclischen Gruppe, ausgewählt aus Indolyl, Isoindolyl, Indolinyl, Benzofuryl, Dihydrobenzofuranyl, Isobenzofuryl, Benzothienyl, Indazolyl, Indoxazinyl, Benzoxazolyl, Anthranilyl, Benzimidazolyl, Benzthiazolyl, Purinyl, Chinolizinyl, Chinolinyl, Isochinolinyl, Tetrahydrochinolinyl, Tetrahydroisochinolinyl, Cinnolinyl, Phthalazinyl, Chinazolinyl, Chinoxalinyl, Naphthyridinyl, Pteridinyl, Indenyl, Isoindenyl, Naphthyl, Tetralinyl, Decalinyl, Benzopyranyl, Pyridopyridiriyl, Benzoxazinyl, Dihydrobenzodioxinyl, Benzodioxolyl, Indanyl, Cyclopentapyridinyl, Pyranopyrrolyl, Chromanyl und Isochromanyl;
wobei die cyclischen Gruppen (a), (b) und (c) unabhängig und gegebenenfalls mit ein, zwei oder drei Substituenten, unabhängig ausgewählt aus Halogen, C₁₋₃-Alkyl, Halogen-substituiertem C₁₋₃-Alkyl, C₁₋₃-Alkoxy, Halogen-substituiertem C₁₋₃-Alkoxy und C₁₋₃-Alkoxy-C₁₋₃-alkyl, substituiert sind;
X unabhängig ausgewählt ist aus Halogen, C₁₋₄-Alkyl und Halogen-substituiertem C₁₋₄-Alkyl;
n 0, 1 oder 2 ist.

4. Verbindung nach Anspruch 3, worin Z Tetrazolyl darstellt; A Methylen darstellt; Q eine cyclische Gruppe, ausgewählt aus Pyridinyl, Phenyl, Furyl, Thienyl, Imidazolyl, Chinolinyl und Isochinolinyl, darstellt, und die cyclische Gruppe gegebenenfalls mit Methyl, Ethyl, Chlor, Trifluormethyl, Methoxy oder Methoxymethyl substituiert ist; X Chlor, Fluor, Methyl oder Trifluormethyl darstellt und n 0 oder 1 ist.

5. Verbindung nach Anspruch 4, worin Z 1,2,3,4-Tetrazolyl darstellt; Q 2- oder 4-Pyridinyl, 3-, 4-, 5- oder 6-Methyl-2-pyridinyl, 4-Ethyl-2-pyridinyl, 4- oder 5-Chlor-2-pyridinyl, 5-Trifluormethyl-2-pyridinyl, 4-Methoxy-2-pyridinyl, 4-Chlorphenyl, 3-Methoxymethyl-2-furyl, 2-Thienyl, 5-Methyl-2-thienyl, 1-Methyl-2-imidazolyl, 3-Isochinolinyl oder 2-Chinolinyl darstellt und X 6-Chlor, 5-Chlor, 6-Trifluormethyl, 5-Trifluormethyl, 5-Fluor, 6-Fluor oder 6-Methyl darstellt.

6. Verbindung nach Anspruch 1, ausgewählt aus
[6-Chlor-3-(1H-1,2,3,4-tetrazol-5-ylmethyl)-1H-indol-2-yl](4-methyl-2-pyridinyl)-methanon;
{6-Chlor-3-[(2-methyl-2H-1,2,3,4-tetrazol-5-yl)methyl]-1H-indol-2-yl}(4-methyl-2-pyridinyl)-methanon;
{6-Chlor-3-[(1-methyl-1H-1,2,3,4-tetrazol-5-yl)methyl]-1H-indol-2-yl}(4-methyl-2-pyridinyl)-methanon;
[6-Chlor-3-(1H-1,2,3,4-tetrazol-5-ylmethyl)-1H-indol-2-yl] (3-methyl-2-pyridinyl)-methanon;
[6-Chlor-3-(1H-1,2,3,4-tetrazol-5-ylmethyl)-1H-indol-2-yl] (5-methyl-2-pyridinyl)-methanon;
[6-Chlor-3-(1H-1,2,3,4-tetrazol-5-ylmethyl)-1H-indol-2-yl] (6-methyl-2-pyridinyl)-methanon;
[6-Chlor-3-(1H-1,2,3,4-tetrazol-5-ylmethyl)-1H-indol-2-yl] (4-ethyl-2-pyridinyl)-methanon;
[6-Chlor-3-(1H-1,2,3,4-tetrazol-5-ylmethyl)-1H-indol-2-yl] (2-pyridinyl)methanon;
[6-Chlor-3-(1H-1,2,3,4-tetrazol-5-ylmethyl)-1H-indol-2-yl](4-chlor-2-pyridinyl)-methanon;
[6-Chlor-3-(1H-1,2,3,4-tetrazol-5-ylmethyl)-1H-indol-2-yl] (5-chlor-2-pyridinyl)methanon;
[6-Chlor-3-(1H-1,2,3,4-tetrazol-5-ylmethyl)-1H-indol-2-yl](5-trifluormethyl-2-pyridinyl)methanon;
[6-Chlor-3-(1H-1,2,3,4-tetrazol-5-ylmethyl)-1H-indol-2-yl] (4-methoxy-2-pyridinyl)-methanon;
[6-Chlor-3-(1H-1,2,3,4-tetrazol-5-ylmethyl)-1H-indol-2-yl] (4-pyridinyl)methanon;
[6-Chlor-3-(1H-1,2,3,4-tetrazol-5-ylmethyl)-1H-indol-2-yl](4-chlorphenyl)-methanon;
[6-Chlor-3-(1H-1,2,3,4-tetrazol-5-ylmethyl)-1H-indol-2-yl] (3-methoxymethyl-2-furyl)methanon;
[6-Chlor-3-(1H-1,2,3,4-tetrazol-5-ylmethyl)-1H-indol-2-yl](2-thienyl)methanon;
[6-Chlor-3-(1H-1,2,3,4-tetrazol-5-ylmethyl)-1H-indol-2-yl](5-methyl-2-thienyl)-methanon;
{6-Chlor-3-[(1-methyl-1H-1,2,3,4-tetrazol-5-yl)methyl]-1H-indol-2-yl}(5-methyl-2-thienyl)methanon;
{6-Chlor-3-[(2-methyl-2H-1,2,3,4-tetrazol-5-yl)methyl]-1H-indol-2-yl}(5-methyl-2-thienyl)methanon;
[6-Chlor-3-(1H-1,2,3,4-tetrazol-5-ylmethyl)-1H-indol-2-yl](1-methyl-2-imidazolyl)-methanon;
[6-Chlor-3-(1H-1,2,3,4-tetrazol-5-ylmethyl)-1H-indol-2-yl](3-isochinolinyl)-methanon;
[6-Chlor-3-(1H-1,2,3,4-tetrazol-5-ylmethyl)-1H-indol-2-yl] (2-chinolinyl)methanon;
[5-Chlor-3-(1H-1,2,3,4-tetrazol-5-ylmethyl)-1H-indol-2-yl](4-methyl-2-pyridinyl)-methanon;
[6-Trifluormethyl-3-(1H-1,2,3,4-tetrazol-5-ylmethyl)-1H-indol-2-yl](4-methyl-2-pyridinyl)methanon;
[6-Trifluormethyl-3-(1H-1,2,3,4-tetrazol-5-ylmethyl)-1H-indol-2-yl](3-isochinolinyl)methanon;
[5-Trifluormethyl-3-(1H-1,2,3,4-tetrazol-5-ylmethyl)-1H-indol-2-yl](4-methyl-2-pyridinyl)methanon;
[5-Fluor-3-(1H-1,2,3,4-tetrazol-5-ylmethyl)-1H-indol-2-yl] (4-methyl-2-pyridinyl)-methanon;
[6-Fluor-3-(1H-1,2,3,4-tetrazol-5-ylmethyl)-1H-indol-2-yl] (4-methyl-2-pyridinyl)-methanon;
[6-Methyl-3-(1H-1,2,3,4-tetrazol-5-ylmethyl)-1H-indol-2-yl] (4-methyl-2-pyridinyl)-methanon;
und die Salze davon.

7. Verbindung nach Anspruch 6, ausgewählt aus:
[6-Chlor-3-(1H-1,2,3,4-tetrazol-5-ylmethyl)-1H-indol-2-yl](4-methyl-2-pyridinyl)-methanon;
{6-Chlor-3-[(2-methyl-2H-1,2,3,4-tetrazol-5-yl)methyl]-1H-indol-2-yl}(4-methyl-2-pyridinyl)methanon;
{6-Chlor-3-[(1-methyl-1H-1,2,3,4-tetrazol-5-yl)methyl]-1H-indol-2-yl}(4-methyl-2-pyridinyl)methanon;
[6-Chlor-3-(1H-1,2,3,4-tetrazol-5-ylmethyl)-1H-indol-2-yl] (5-methyl-2-pyridinyl)-methanon;
[6-Chlor-3-(1H-1,2,3,4-tetrazol-5-ylmethyl)-1H-indol-2-yl] (4-ethyl-2-pyridinyl)-methanon;
[6-Chlor-3-(1H-1,2,3,4-tetrazol-5-ylmethyl)-1H-indol-2-yl] (4-chlor-2-pyridinyl)-methanon;
[6-Chlor-3-(1H-1,2,3,4-tetrazol-5-ylmethyl)-1H-indol-2-yl] (5-chlor-2-pyridinyl)-methanon;
[6-Chlor-3-(1H-1,2,3,4-tetrazol-5-ylmethyl)-1H-indol-2-yl](4-methoxy-2-pyridinyl)-methanon;
[6-Chlor-3-(1H-1,2,3,4-tetrazol-5-ylmethyl)-1H-indol-2-yl](5-methyl-2-thienyl)-methanon;
{6-Chlor-3-[(1-methyl-1H-1,2,3,4-tetrazol-5-yl)methyl]-1H-indol-2-yl}(5-methyl-2-thienyl)methanon;
{6-Chlor-3-[(2-methyl-2H-1,2,3,4-tetrazol-5-yl)methyl]-1H-indol-2-yl}(5-methyl-2-thienyl)methanon;
[6-Chlor-3-(1H-1,2,3,4-tetrazol-5-ylmethyl)-1H-indol-2-yl] (3-isochinolinyl)-methanon;
[6-Trifluormethyl-3-(1H-1,2,3,4-tetrazol-5-ylmethyl)-1H-indol-2-yl](3-isochinolinyl)-methanon;
[5-Trifluormethyl-3-(1H-1,2,3,4-tetrazol-5-ylmethyl)-1H-indol-2-yl](4-methyl-2-pyridinyl)methanon;
[5-Fluor-3-(1H-1,2,3,4-tetrazol-5-ylmethyl)-1H-indol-2-yl](4-methyl-2-pyridinyl)-methanon;
[6-Fluor-3-(1H-1,2,3,4-tetrazol-5-ylmethyl)-1H-indol-2-yl](4-methyl-2-pyridinyl)-methanon;
und die Salze davon.

8. Pharmazeutische Zusammensetzung, verwendbar für die Behandlung eines medizinischen Zustands, in den Prostaglandine als Pathogene einbezogen sind, die eine Verbindung der Formel (I) von Anspruch 1 und einen pharmazeutisch inerten Träger umfasst.

9. Verwendung einer Verbindung nach Anspruch 1 zur Herstellung eines Arzneimittels für die Behandlung eines medizinischen Zustands, in den Prostaglandine als Pathogene einbezogen sind, bei einem Säuger.

## Revendications

1. Composé répondant la formule suivante : 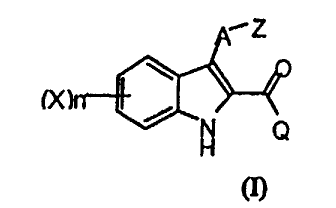 ou ses sels pharmaceutiquement acceptables, formule dans laquelle
Z représente un groupe tétrazolyle facultativement substitué avec un substituant alkyle en C₁ à C₄ ou alkyle en C₁ à C₄ halogéno-substitué ;
A représente un groupe alkylène en C₁ à C₆ ;
Q représente un groupe cyclique choisi entre
(a) un groupe phényle ;
(b) un groupe monocyclique penta- ou hexagonal partiellement saturé, totalement saturé ou totalement insaturé comprenant un à quatre hétéroatomes choisis indépendamment entre N, O et S ; et
(c) un groupe bicyclique consistant en deux noyaux penta- à octogonaux condensés, partiellement saturés, totalement saturés ou totalement insaturés, indépendamment, et comprenant facultativement un à quatre hétéroatomes choisis indépendamment entre N, O et S ;
lesdits groupes cycliques (a), (b) et (c) étant indépendamment et facultativement substitués avec un à cinq substituants choisis indépendamment entre les substituants halogéno, alkyle en C₁ à C₄, alkyle en C₁ à C₄ halogéno-substitué, OH, alkoxy en C₁ à C₄, alkoxy en C₁ à C₄ halogéno-substitué, alkylthio en C₁ à C₄, NO₂, NH₂, mono- ou di(alkyle en C₁ à C₄)amino, CN, hydroxy-alkyle en C₁ à C₄ et (alkoxy en C₁ à C₄) (alkyle en C₁ à C₄) ;
X est choisi indépendamment entre des groupes halogéno, alkyle en C₁ à C₄, alkyle en C₁ à C₄ halogéno-substitué, OH, alkoxy en C₁ à C₄, alkoxy en C₁ à C₄ halogéno-substitué, alkylthio en C₁ à C₄, NO₂, NH₂ et mono- ou di(alkyle en C₁ à C₄) amino et CN ; et
n est égal à O, 1, 2, 3 ou 4.

2. Composé suivant la revendication 1, dans lequel
Z représente un groupe tétrazolyle facultativement substitué avec un groupe alkyle en C₁ à C₃ ou alkyle en C₁ à C₃ halogéno-substitué ;
A représente un groupe alkylène en C₁ à C₅ ;
Q représente un groupe cyclique choisi entre :
(a) un groupe phényle ;
(b) un groupe monocyclique penta- ou hexagonal partiellement saturé, totalement saturé ou totalement insaturé comprenant un à trois hétéroatomes choisis indépendamment entre N, O et S ; et
(c) un groupe bicyclique consistant en deux noyaux penta- ou hexagonaux condensés, partiellement saturés, totalement saturés ou totalement insaturés, indépendamment, et comprenant facultativement un à trois hétéroatomes choisis indépendamment entre N, O et S ;
lesdits groupes cycliques (a), (b) et (c) étant indépendamment et facultativement substitués avec un, deux ou trois substituants choisis indépendamment entre des substituants halogéno, alkyle en C₁ à C₄, alkyle en C₁ à C₄ halogéno-substitué, alkoxy en C₁ à C₄, alkoxy en C₁ à C₄ halogéno-substitué et (alkoxy en C₁ à C₄) (alkyle en C₁ à C₄) ;
X est choisi indépendamment entre des groupes halogéno, alkyle en C₁ à C₄, alkyle en C₁ à C₄ halogéno-substitué, OH et alkoxy en C₁ à C₄ ; et
n est égal à O, 1, 2 ou 3.

3. Composé suivant la revendication 1, dans lequel
Z représente un groupe tétrazolyle ;
A représente un groupe alkylène en C₁ à C₄ ;
Q est choisi entre les groupes suivants :
(a) un groupe phényle ;
(b) un groupe monocyclique pentagonal choisi entre les groupes furyle, thiényle, pyrrolyle, pyrrolinyle, pyrrolidinyle, dioxolanyle, oxazolyle, thiazolyle, imidazolyle, imidazolinyle, imidazolidinyle, pyrazolyle, pyrazolinyle, pyrazolidinyle, isoxazolyle, isothiazolyle, dithiolyle, oxathiolyle, oxadiazolyle, thiadiazolyle, oxatriazolyle, dioxazolyle, oxathiazolyle et oxathiolyle, et un groupe monocyclique hexagonal choisi entre les groupes pyrannyle, pyridinyle, pipéridinyle, dioxinyle, dioxanyle, morpholinyle, dithianyle, thiomorpholinyle, pyridazinyle, pyrimidinyle, pyrazinyle, pipérazinyle, triazinyle, triazinyle, trithianyle, oxazinyle, oxathiazinyle, isoxazinyle et oxadiazinyle ; et
(c) un groupe bicyclique choisi entre les groupes indolyle, iso-indolyle, indolinyle, benzofuryle, dihydrobenzofurannyle, isobenzofuryle, benzo-thiényle, indazolyle, indoxazinyle, benzoxazolyle, anthranilyle, benzimidazolyle, benzthiazolyle, purinyle, quinolizinyle, quinolinyle, isoquinolinyle, tétrahydro-quinolinyle, tétrahydro-isoquinolinyle, cinnolinyle, phtalazinyle, quinazolinyle, quinoxalinyle, naphtyridinyle, ptéridinyle, indényle, iso-indényle, naphtyle, tétralinyle, décalinyle, benzopyrannyle, pyridopyridinyle, benzoxazinyle, dihydrobenzodioxinyle, benzodioxolyle, indanyle, cyclopentapyridinyle, pyrannopyrrolyle, chromanyle et isochromanyle ;
lesdits groupes cycliques (a), (b) et (c) étant indépendamment et facultativement substitués avec un, deux ou trois substituants choisis indépendamment entre des substituants halogéno, alkyle en C₁ à C₃, alkyle en C₁ à C₃ halogéno-substitué, alkoxy en C₁ à C₃, alkoxy en C₁ à C₃ halogéno-substitué et (alkoxy en C₁ à C₃) (alkyle en C₁ à C₃) ;
X est choisi indépendamment entre des groupes halogéno, alkyle en C₁ à C₄ et alkyle en C₁ à C₄ halogéno-substitué ; et
n est égal à O, 1 ou 2.

4. Un composé suivant la revendication 3, dans lequel Z représente un groupe tétrazolyle ; A représente un groupe méthylène ; Q représente un groupe cyclique choisi entre les groupes pyridinyle, phényle, furyle, thiényle, imidazolyle, quinolinyle et isoquinolinyle, et le groupe cyclique est facultativement substitué avec des substituants méthyle, éthyle, chloro, trifluorométhyle, méthoxy ou méthoxyméthyle ; X représente un groupe chloro, fluoro, méthyle ou trifluorométhyle ; et n est égal à 0 ou 1.

5. Un composé suivant la revendication 4, dans lequel Z représente un groupe 1,2,3,4-tétrazolyle ; Q représente un groupe 2- ou 4-pyridinyle, 3-, 4-, 5- ou 6-méthyl-2-pyridinyle, 4-éthyl-2-pyridinyle, 4- ou 5-chloro-2-pyridinyle, 5-trifluorométhyl-2-pyridinyle, 4-méthoxy-2-pyridinyle, 4-chlorophényle, 3-méthoxyméthyl-2-furyle, 2-thiényle, 5-méthyl-2-thiényle, 1-méthyl-2-imidazolyle, 3-isoquinolinyle ou 2-quinolinyle ; X représente un groupe 6-chloro, 5-chloro, 6-trifluorométhyle, 5-trifluorométhyle, 5-fluoro, 6-fluoro ou 6-méthyle.

6. Composé suivant la revendication 1, choisi entre les suivants
[6-chloro-3-(1H-1,2,3,4-tétrazole-5-ylméthyl)-1H-indole-2-yl](4-méthyl-2-pyridinyl)méthanone ;
{6-chloro-3-[(2-méthyl-2H-1,2,3,4-tétrazole-5-yl)méthyl]-1H-indole-2-yl}(4-méthyl-2-pyridinyl)méthanone ;
6-chloro-3-[(1-méthyl-1H-1,2,3,4-tétrazole-5-yl)méthyl]-1H-indole-2-yl}(4-méthyl-2-pyridinyl)méthanone ;
[6-chloro-3-(1H-1,2,3,4-tétrazole-5-ylméthyl)-1H-indole-2-yl](3-méthyl-2-pyridinyl)méthanone ;
[6-chloro-3-(1H-1,2,3,4-tétrazole-5-ylméthyl)-1H-indole-2-yl] (5-méthyl-2-pyridinyl)méthanone ;
[6-chloro-3-(1H-1,2,3,4-tétrazole-5-ylméthyl)-1H-indole-2-yl](6-méthyl-2-pyridinyl)méthanone ;
[6-chloro-3-(1H-1,2,3,4-tétrazole-5-ylméthyl)-1H-indole-2-yl](4-éthyl-2-pyridinyl)méthanone ;
[6-chloro-3-(1H-1,2,3,4-tétrazole-5-ylméthyl)-1H-indole-2-yl] (2-pyridinyl)méthanone ;
[6-chloro-3-(1H-1,2,3,4-tétrazole-5-ylméthyl)-1H-indole-2-yl] (4-chloro-2-pyridinyl)méthanone ;
[6-chloro-3-(1H-1,2,3,4-tétrazole-5-ylméthyl)-1H-indole-2-yl] (5-chloro-2-pyridinyl)méthanone ;
[6-chloro-3-(1H-1,2,3,4-tétrazole-5-ylméthyl)-1H-indole-2-yl](5-trifluorométhyl-2-pyridinyl)méthanone ;
[6-chloro-3-(1H-1,2,3,4-tétrazole-5-ylméthyl)-1H-indole-2-yl] (4-méthoxy-2-pyridinyl)méthanone ;
[6-chloro-3-(1H-1,2,3,4-tétrazole-5-ylméthyl)-1H-indole-2-yl] (4-pyridinyl)méthanone ;
[6-chloro-3-(1H-1,2,3,4-tétrazole-5-ylméthyl)-1H-indole-2-yl](4-chlorophényl)méthanone ;
[6-chloro-3-(1H-1,2,3,4-tétrazole-5-ylméthyl)-1H-indole-2-yl](3-méthoxyméthyl-2-furyl)méthanone ;
[6-chloro-3-(1H-1,2,3,4-tétrazole-5-ylméthyl)-1H-indole-2-yl](2-thiényl)méthanone ;
[6-chloro-3-(1H-1,2,3,4-tétrazole-5-ylméthyl)-1H-indole-2-yl](5-méthyl-2-thiényl)méthanone ;
{6-chloro-3-[(1-méthyl-1H-1,2,3,4-tétrazole-5-yl)méthyl]-1H-indole-2-yl}(5-méthyl-2-thiényl)méthanone ;
{6-chloro-3-[(2-méthyl-2H-1,2,3,4-tétrazole-5-yl)méthyl]-1H-indole-2-yl}(5-méthyl-2-thiényl)méthanone ;
[6-chloro-3-(1H-1,2,3,4-tétrazole-5-ylméthyl)-1H-indole-2-yl] (1-méthyl-2-imidazolyl)méthanone ;
[6-chloro-3-(1H-1,2,3,4-tétrazole-5-ylméthyl)-1H-indole-2-yl] (3-isoquinolinyl)méthanone ;
[6-chloro-3-(1H-1,2,3,4-tétrazole-5-ylméthyl)-1H-indole-2-yl](2-quinolinyl)méthanone ;
[5-chloro-3-(1H-1,2,3,4-tétrazole-5-ylméthyl)-1H-indole-2-yl] (4-méthyl-2-pyridinyl)méthanone ;
[6-trifluorométhyl-3-(1H-1,2,3,4-tétrazole-5-ylméthyl)-1H-indole-2-yl] (4-méthyl-2-pyridinyl)méthanone ;
[6-trifluorométhyl-3-(1H-1,2,3,4-tétrazole-5-ylméthyl)-1H-indole-2-yl] (3-isoquinolinyl)méthanone ;
[5-trifluorométhyl-3-(1H-1,2,3,4-tétrazole-5-ylméthyl)-1H-indole-2-yl] (4-méthyl-2-pyridinyl)méthanone ;
[5-fluoro-3-(1H-1,2,3,4-tétrazole-5-ylméthyl)-1H-indole-2-yl] (4-méthyl-2-pyridinyl)méthanone ;
[6-fluoro-3-(1H-1,2,3,4-tétrazole-5-ylméthyl)-1H-indole-2-yl](4-méthyl-2-pyridinyl)méthanone ;
[6-méthyl-3-(1H-1,2,3,4-tétrazole-5-ylméthyl)-1H-indole-2-yl] (4-méthyl-2-pyridinyl)méthanone,
et ses sels.

7. Composé suivant la revendication 6, choisi entre les suivants
[6-chloro-3-(1H-1,2,3,4-tétrazole-5-ylméthyl)-1H-indole-2-yl](4-méthyl-2-pyridinyl)méthanone ;
{6-chloro-3-[(2-méthyl-2H-1,2,3,4-tétrazole-5-yl)méthyl]-1H-indole-2-yl}(4-méthyl-2-pyridinyl)méthanone ;
6-chloro-3-[(1-méthyl-1H-1,2,3,4-tétrazole-5-yl)méthyl]-1H-indole-2-yl}(4-méthyl-2-pyridinyl)méthanone ;
[6-chloro-3-(1H-1,2,3,4-tétrazole-5-ylméthyl)-1H-indole-2-yl] (5-méthyl-2-pyridinyl)méthanone ;
[6-chloro-3-(1H-1,2,3,4-tétrazole-5-ylméthyl)-1H-indole-2-yl] (4-éthyl-2-pyridinyl)méthanone ;
[6-chloro-3-(1H-1,2,3,4-tétrazole-5-ylméthyl)-1H-indole-2-yl](4-chloro-2-pyridinyl)méthanone ;
[6-chloro-3-(1H-1,2,3,4-tétrazole-5-ylméthyl)-1H-indole-2-yl](5-chloro-2-pyridinyl)méthanone ;
[6-chloro-3-(1H-1,2,3,4-tétrazole-5-ylméthyl)-1H-indole-2-yl](4-méthoxy-2-pyridinyl)méthanone ;
[6-chloro-3-(1H-1,2,3,4-tétrazole-5-ylméthyl)-1H-indole-2-yl](5-méthyl-2-thiényl)méthanone ;
{6-chloro-3-[(1-méthyl-1H-1,2,3,4-tétrazole-5-yl)méthyl]-1H-indole-2-yl}(5-méthyl-2-thiényl)méthanone ;
{6-chloro-3 -[(2-méthyl-2H-1,2,3,4-tétrazole-5-yl)méthyl]-1H-indole-2-yl}(5-méthyl-2-thiényl)méthanone ;
[6-chloro-3-(1H-1,2,3,4-tétrazole-5-ylméthyl)-1H-indole-2-yl](3-isoquinolinyl)méthanone ;
[6-trifluorométhyl-3-(1H-1,2,3,4-tétrazole-5-ylméthyl)-1H-indole-2-yl](3-isoquinolinyl)méthanone ;
[5-trifluorométhyl-3-(1H-1,2,3,4-tétrazole-5-ylméthyl)-1H-indole-2-yl] (4-méthyl-2-pyridinyl)méthanone ;
[5-fluoro-3-(1H-1,2,3,4-tétrazole-5-ylméthyl)-1H-indole-2-yl](4-méthyl-2-pyridinyl)méthanone ;
[6-fluoro-3-(1H-1,2,3,4-tétrazole-5-ylméthyl)-1H-indole-2-yl] (4-méthyl-2-pyridinyl)méthanone ;
et ses sels.

8. Composition pharmaceutique utile pour le traitement d'un état médical dans lequel des prostaglandines sont impliquées comme agents pathogènes, qui comprend un composé de formule (I) suivant la revendication 1 et un support pharmaceutiquement inerte.

9. Utilisation d'un composé suivant la revendication 1 pour la production d'un médicament destiné au traitement d'un état médical chez un mammifère dans lequel des prostaglandines sont impliquées comme agents pathogènes.
